# EUROPEAN PATENT APPLICATION

(11) **EP 4 585 588 A1**
(43) Date of publication of application: **16.07.2025**
(21) Application number: 23862055.3
(22) Date of filing: 19.07.2023
(51) Int. Cl.: C07D 235/08, C07D 235/10, C07D 235/12, C07D 235/14, A61K 31/4184, A61P 9/04

(54) **BENZIMIDAZOLE COMPOUND, AND PREPARATION METHOD THEREFOR AND USE THEREOF**

(30) Priority: 09.09.2022 CN 202211104193
(71) Applicant: Suzhou Pharmavan Co., Ltd., Suzhou, Jiangsu 215000 (CN)
(72) Inventor: YANG, Fei, Suzhou, Jiangsu 215000 (CN); HUANG, Ruohe, Suzhou, Jiangsu 215000 (CN); LI, Ling, Suzhou, Jiangsu 215000 (CN); YU, Yunhui, Suzhou, Jiangsu 215000 (CN); ZHANG, Yu, Suzhou, Jiangsu 215000 (CN); WU, Wenping, Suzhou, Jiangsu 215000 (CN); SUN, Yanan, Suzhou, Jiangsu 215000 (CN); CAO, Yu, Suzhou, Jiangsu 215000 (CN); FENG, Haimei, Suzhou, Jiangsu 215000 (CN)
(74) Representative: Plougmann Vingtoft a/s
(86) International application number: PCT/CN2023/108143
(87) International publication number: WO 2024/051354

(57) **Abstract**

Provided in the present application are a benzimidazole compound, and a preparation method therefor and the use thereof, wherein the benzimidazole compound has a structure as shown in formula A, has an effect of treating chronic heart failure, and has broad application prospects.

## Description

### TECHNICAL FIELD

The present application belongs to the technical field of chemical medicines and relates to a benzimidazole compound, a preparation method therefor and use thereof.

### BACKGROUND

Heart failure is a clinical syndrome; its typical symptoms (such as dyspnea, ankle swelling and fatigue) may be accompanied by signs (such as increased jugular venous pressure, lung crackle and peripheral edema) caused by structural and/or functional cardiac abnormalities, resulting in a reduced cardiac output and/or an increased intracardiac pressure during resting or pressure. Heart failure occurs only when symptoms are apparent.

Existing drugs are mainly used for alleviating symptoms caused by triggering compensatory mechanisms based on a sympathetic adrenergic system and the like during heart failure. The sympathetic adrenergic system, the renin-angiotensin-aldosterone (RAA) system and the release of vasoactive peptide (the natriuretic peptide) are regulated by myocardial injury stimulation, and some myocardial functions are compensated by increasing preload (edema), afterload reperfusion (vasoconstriction) and cardiomyocyte calcium modulation (myocardial hypertrophy). In general, the first-line treatment regimen is still unable to prevent the progression of heart failure.

Therefore, in the art, the development of drugs for effectively treating chronic heart failure is the focus of research, including heart failure with preserved ejection fraction and heart failure with reduced ejection fraction.

### SUMMARY

The present application provides a benzimidazole compound, a preparation method therefor and use thereof.

In a first aspect, the present application provides a benzimidazole compound. The benzimidazole compound has a structure represented by the following Formula A: 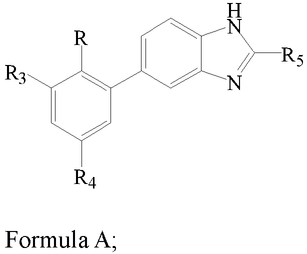
wherein R is hydroxyl or 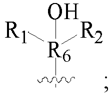
R₁, R₂, R₃ and R₅ are each independently hydrogen, halogen, nitro, amino, hydroxyl, cyano, carboxyl, C1-C4 linear or branched alkoxyformyl, aminoformyl, aminoformyl substituted on N with C1-C4 linear or branched alkyl, C1-C4 linear or branched alkyl, C1-C4 alkoxy and C1-C4 alkylamino;
R₄ is fluorine, bromine, nitro, hydroxyl, cyano, carboxyl, alkoxyformyl, aminoformyl, N-substituted aminoformyl, C1-C4 linear or branched alkyl, non-halogenated C1-C4 alkoxy or C1-C4 alkylamino; and
R₆ is C1-C4 linear or branched alkylene.

The benzimidazole compound having the structure represented by Formula A of the present application has an effect of treating chronic heart failure.

In some preferred embodiments, the benzimidazole compound has a structure represented by Formula I or Formula II: 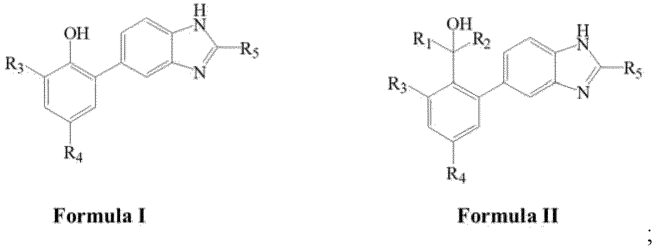 wherein R₁, R₂, R₃, R₄ and R₅ are defined in the same manner as those in Formula A.

In some preferred embodiments, R₁, R₂, R₃ and R₅ are each independently any one of hydrogen, fluorine, chlorine, bromine, amino, hydroxyl, cyano, methoxyformyl, ethoxyformyl, dimethylaminoformyl, diethylaminoformyl, trifluoromethyl, methyl, ethyl, n-propyl, isopropyl, allyl, cyanomethyl, methoxy, trifluoromethoxy, ethoxy, methylamino, dimethylamino, ethylamino or diethylamino.

In some preferred embodiments, R₄ is any one of fluorine, bromine, amino, hydroxyl, cyano, carboxyl, methoxyformyl, ethoxyformyl, aminoformyl, dimethylaminoformyl, diethylaminoformyl, trifluoromethyl, methyl, ethyl, n-propyl, isopropyl, allyl, cyanomethyl, methoxy, ethoxy, methylamino, dimethylamino, ethylamino or diethylamino.

In some more preferred embodiments, R₁ and R₂ are hydrogen or methyl.

In some more preferred embodiments, R₃ is cyano, fluorine or hydrogen.

In some more preferred embodiments, R₄ is fluorine, allyl, n-propyl, cyanomethyl, carboxyl, methoxyformyl, aminoformyl or dimethylaminoformyl.

In some more preferred embodiments, R₅ is hydrogen or methyl.

In the present application, where the number of carbon atoms is used to limit a group, for example, C1-C4 linear or branched alkyl and C1-C4 alkoxy, and the limited number of carbon atoms refers to all integer values within a limited range; for example, C1-C4 means that the number of carbon atoms may be 1, 2, 3 or 4.

In some preferred embodiments, the benzimidazole compound is any one of compounds represented by the following Formula I-1 to Formula I-47 and Formula II-1 to Formula II-144:

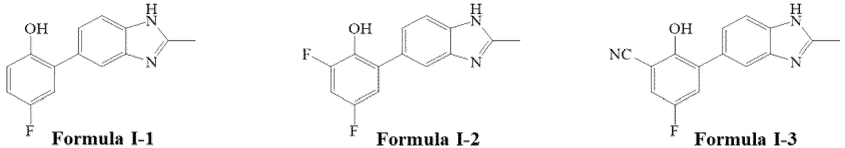

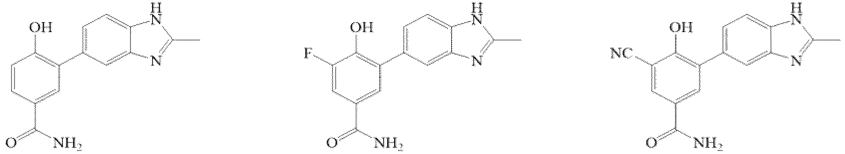

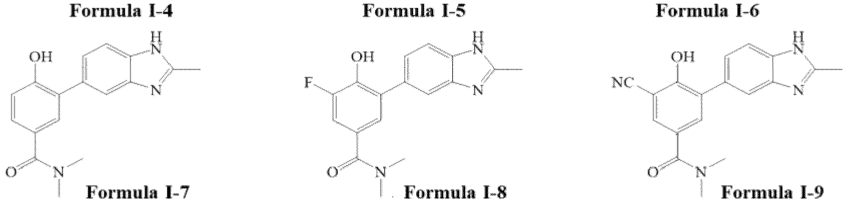

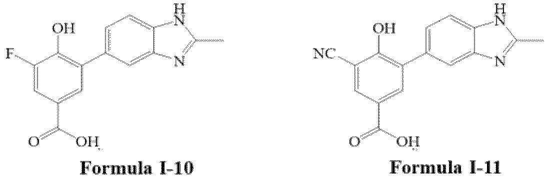

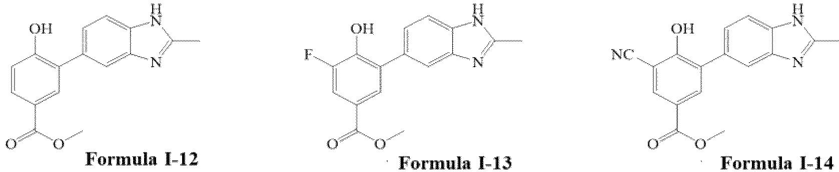

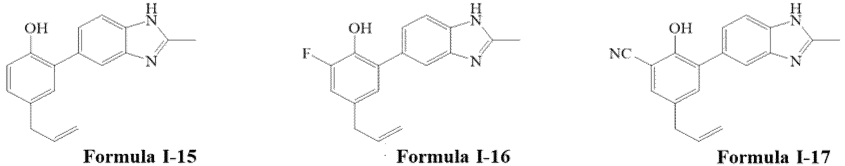

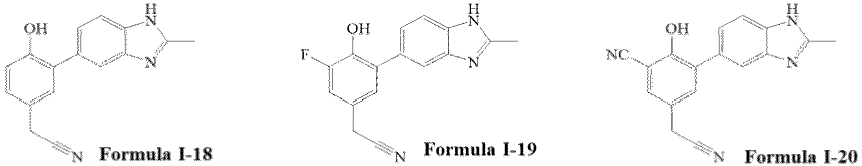

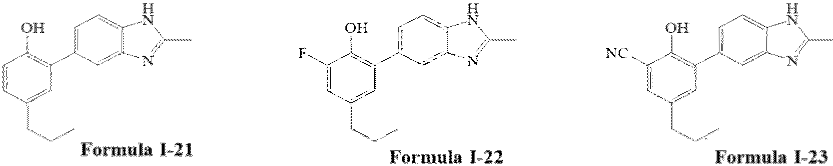

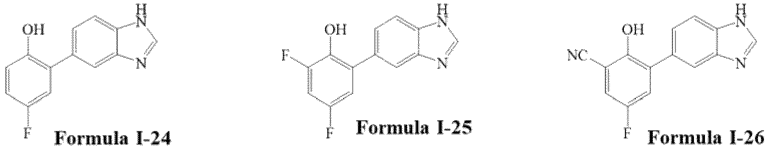

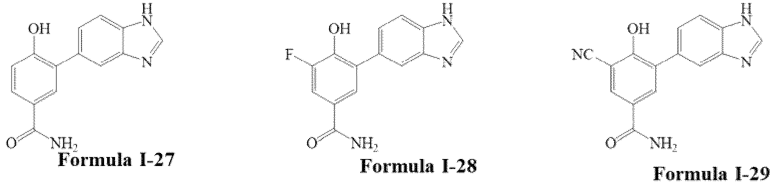

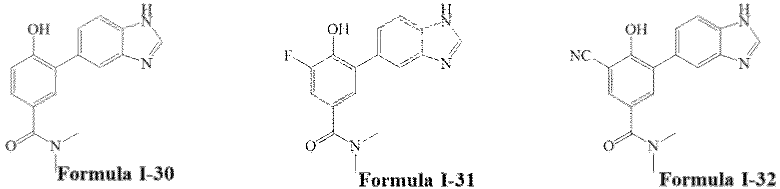

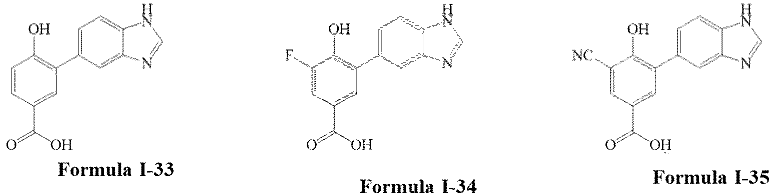

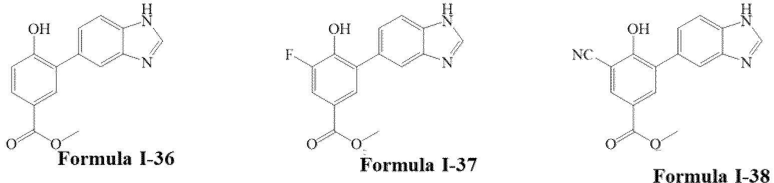

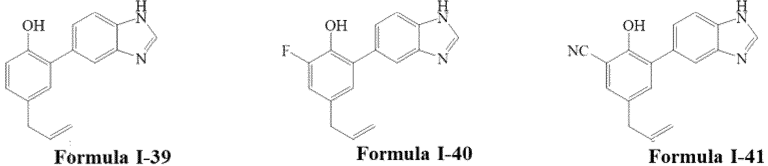

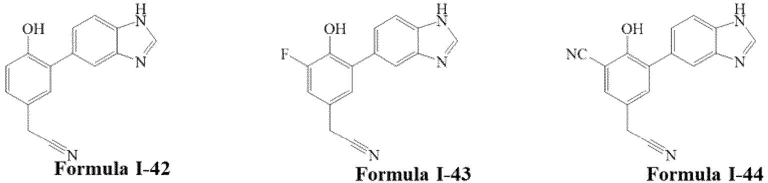

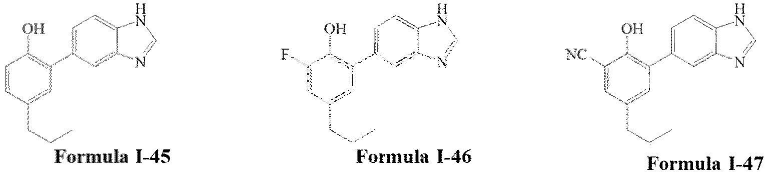

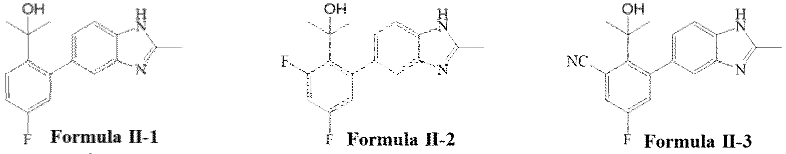

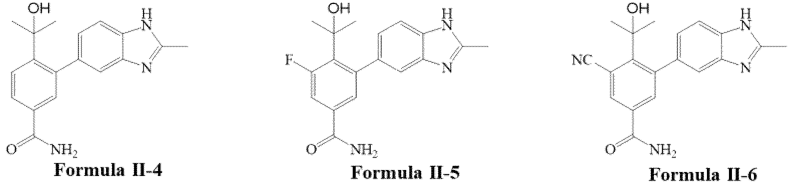

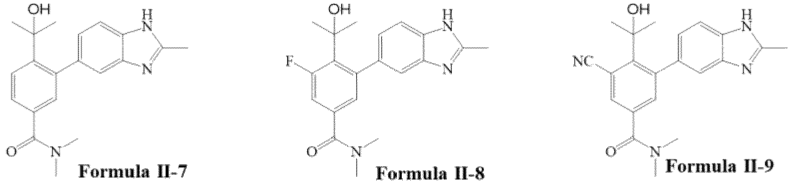

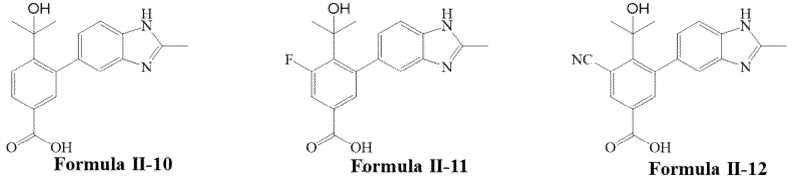

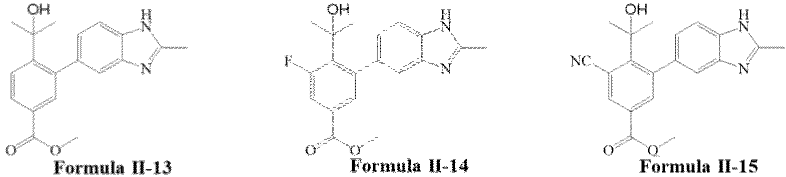

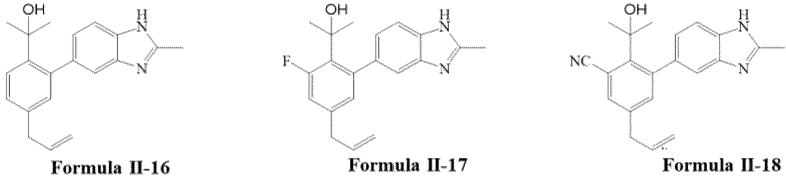

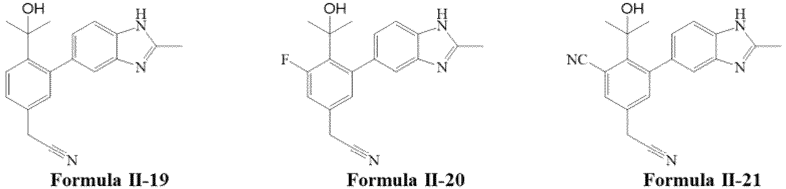

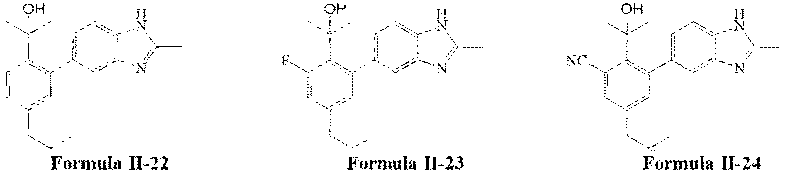

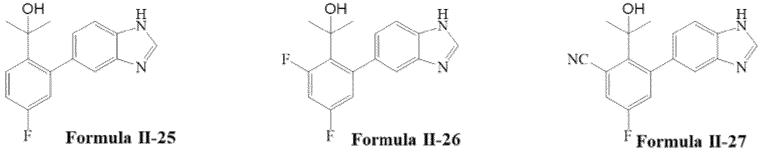

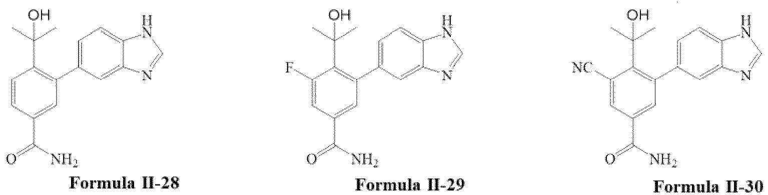

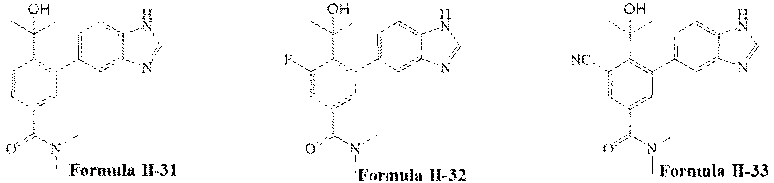

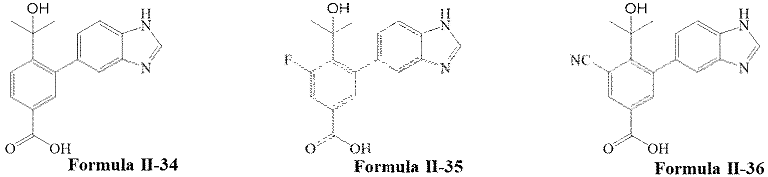

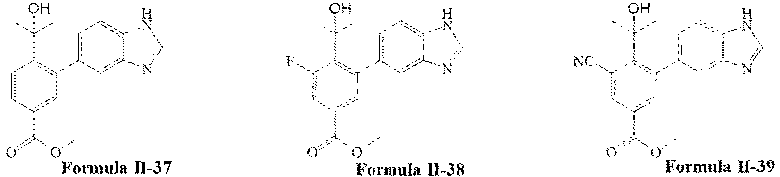

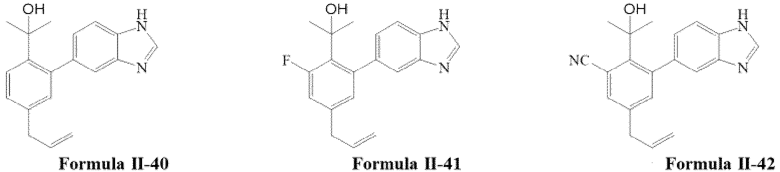

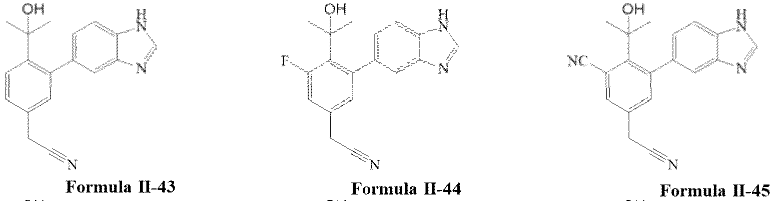

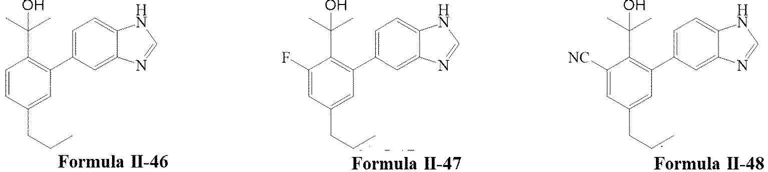

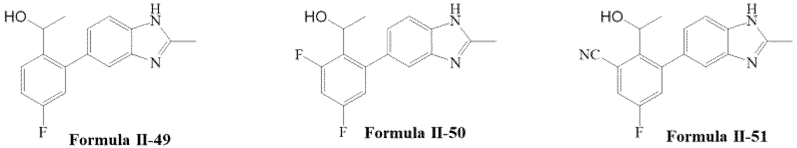

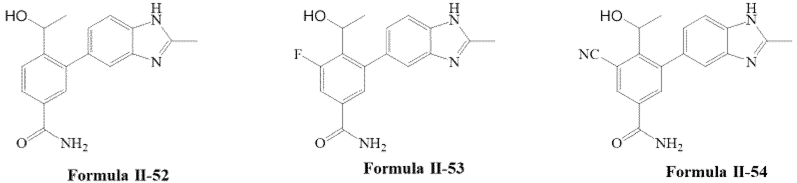

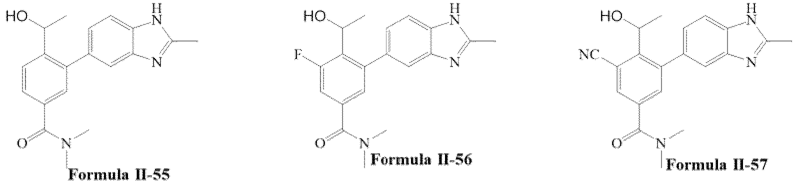

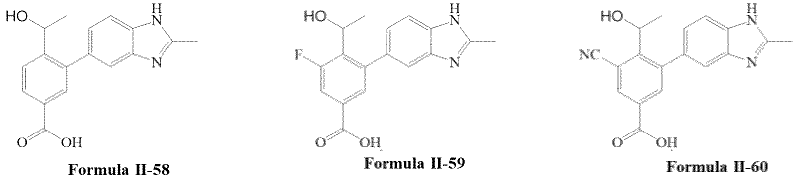

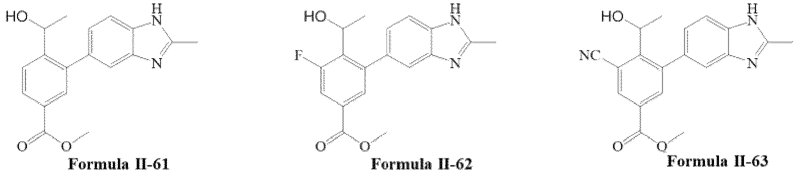

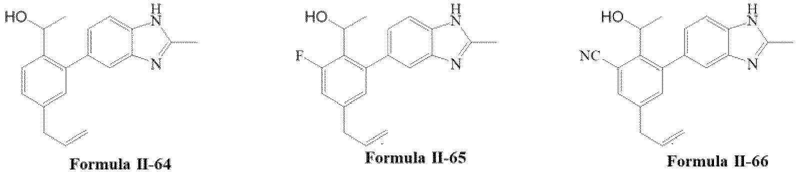

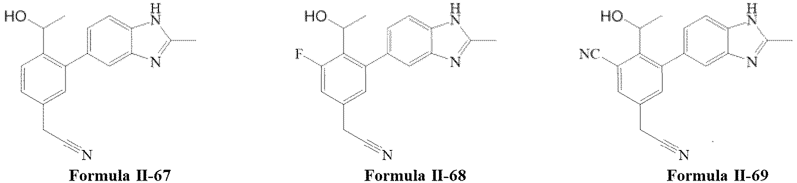

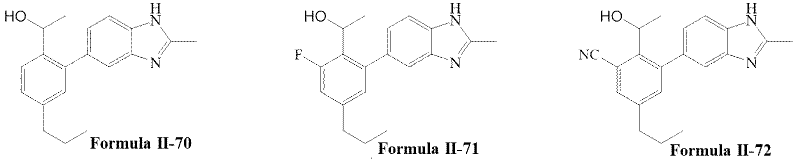

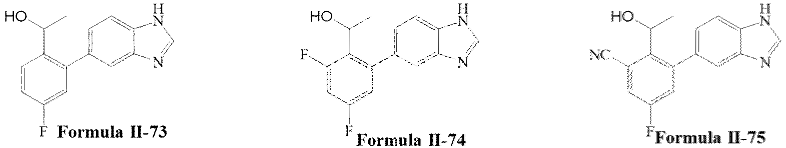

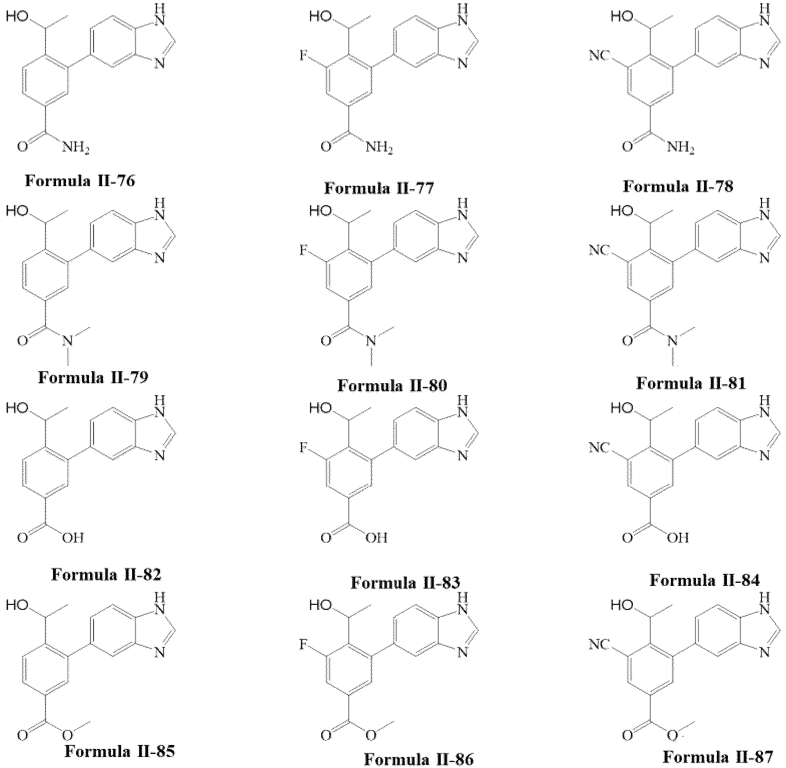

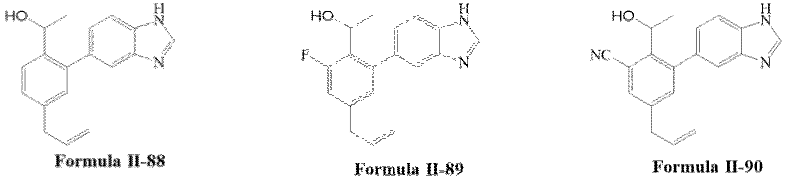

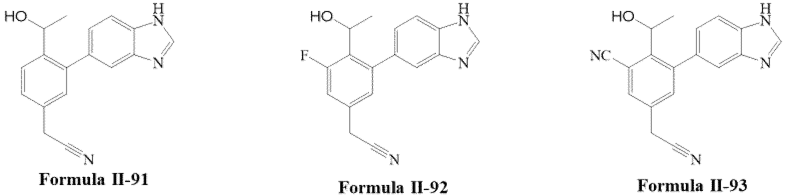

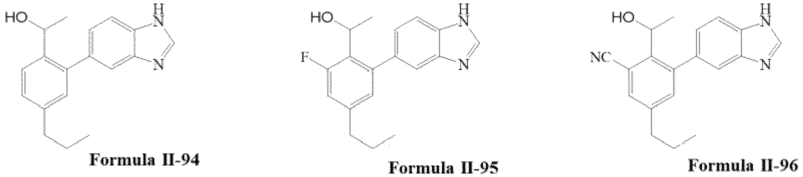

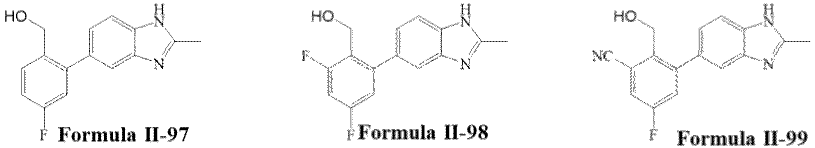

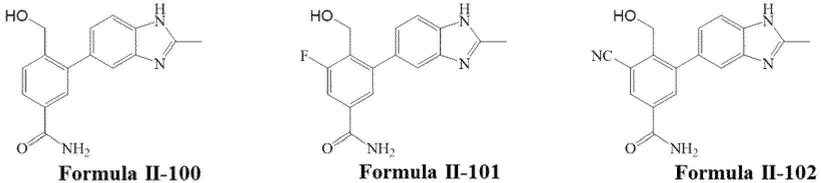

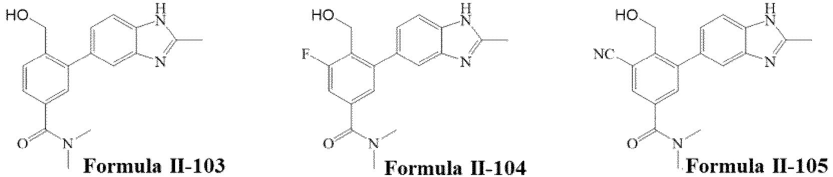

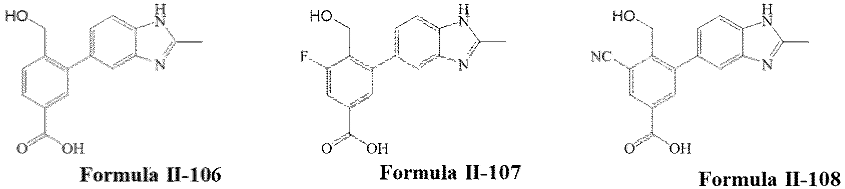

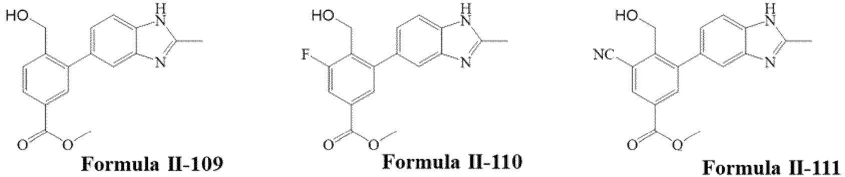

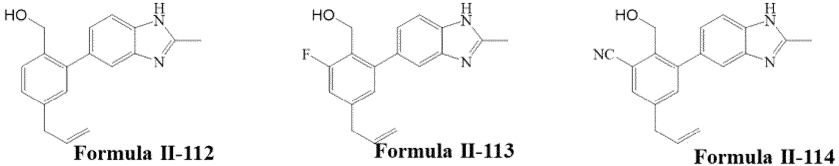

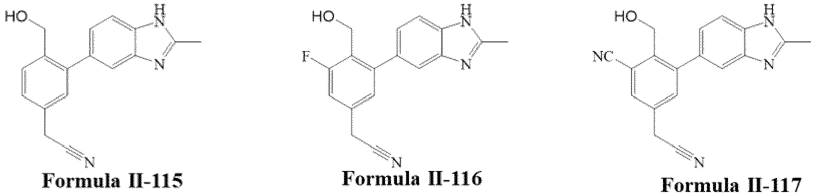

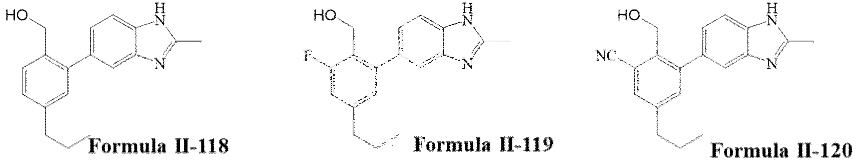

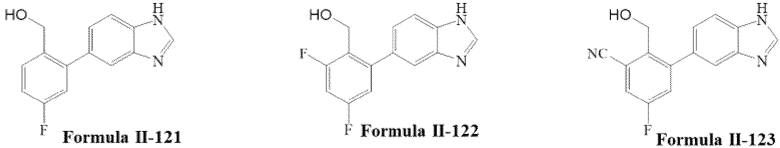

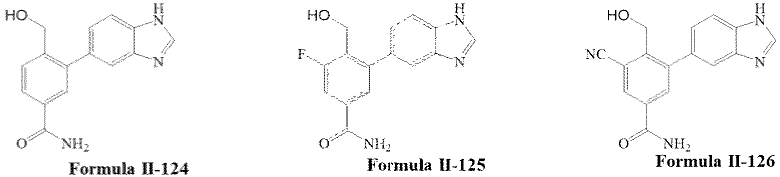

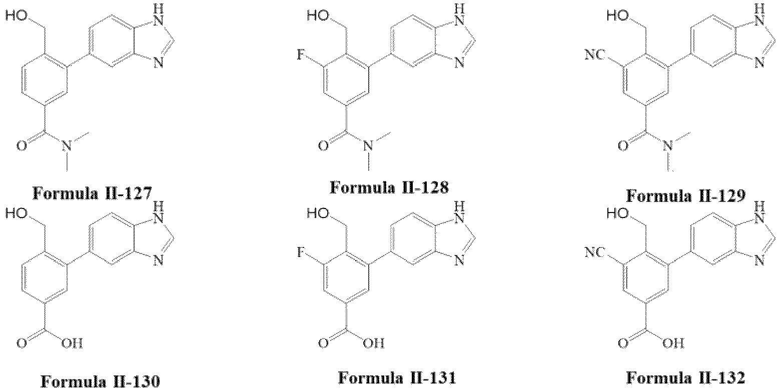

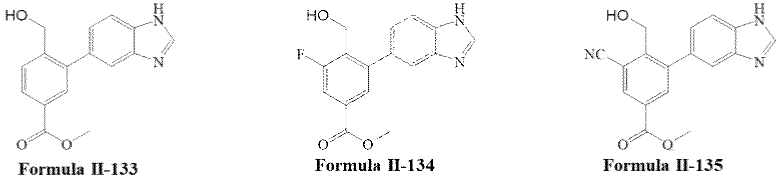

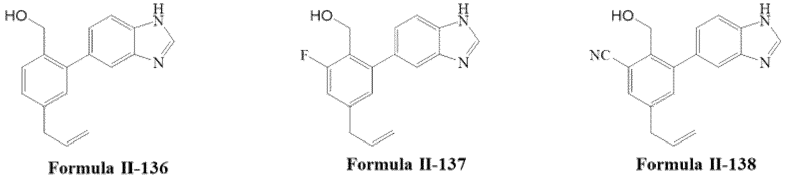

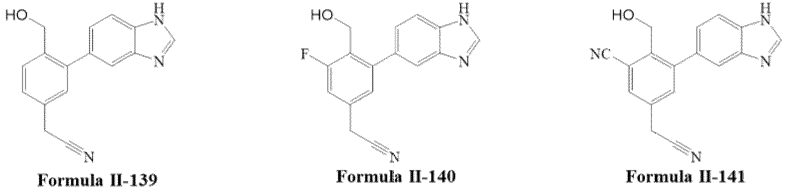

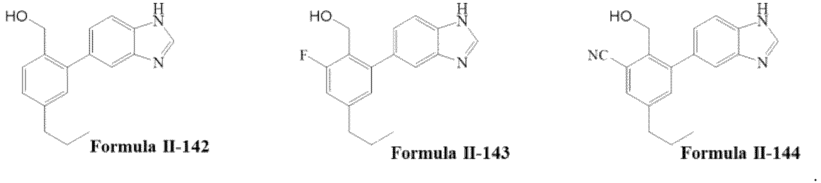

In a second aspect, the present application provides a method for preparing the benzimidazole compound described above. The preparation method includes the following step: performing a Suzuki reaction on a brominated compound represented by Formula B and a boronic acid compound represented by Formula V under an action of a catalyst to obtain the benzimidazole compound represented by Formula A with the following reaction formula: 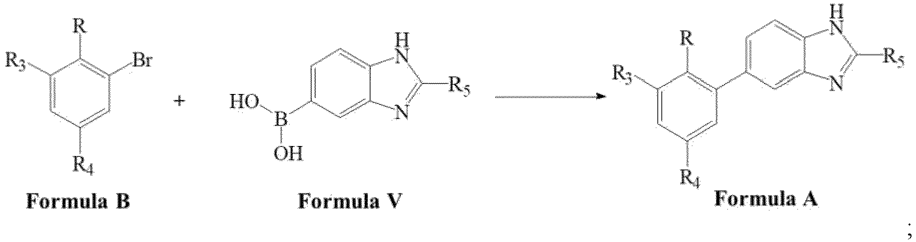 wherein R, R₁, R₂, R₃, R₄ and R₅ are defined in the same manner as those described above, which is not repeated here.

Preferably, a molar ratio of the brominated compound represented by Formula B to the boronic acid compound represented by Formula V is 1:0.8 to 1:3, for example, 0.8:3, 0.85:3, 0.88:3, 0.9:3, 0.95:3 or 1:3.

Preferably, the catalyst is any one or a combination of at least two of tetrakis(triphenylphosphine)palladium, [1,1-bis(diphenylphosphino)ferrocene]palladium(II) dichloride or palladium acetate.

Preferably, a molar ratio of the catalyst to the boronic acid compound represented by Formula V is 0.001:1 to 0.5:1, for example, 0.001:1, 0.005:1, 0.008:1, 0.1:1, 0.2:1, 0.3:1, 0.4:1 or 0.5:1.

Preferably, the Suzuki reaction is conducted in the presence of an alkaline substance.

Preferably, the alkaline substance is any one or a combination of at least two of potassium fluoride, potassium acetate, sodium carbonate, potassium carbonate or potassium phosphate.

Preferably, the Suzuki reaction is conducted in an organic solvent, and the organic solvent is any one or a combination of at least two of DMF, toluene, ethanol, 1,4-dioxane, water or THF.

Preferably, the Suzuki reaction is conducted for a period of 0.25-48 h (for example, 0.25 h, 0.5 h, 1 h, 3 h, 8 h, 10 h, 13 h, 15 h, 18 h, 20 h, 24h, 28 h, 33 h, 38 h, 40 h, 42 h, 45 h or 48 h) at a temperature of 70-150 °C (for example, 70 °C, 75 °C, 80 °C, 90 °C, 100 °C, 110 °C, 120 °C, 130 °C, 140 °C or 150 °C).

In a third aspect, the present application provides a pharmaceutically acceptable salt of the benzimidazole compound described above.

In the present application, the pharmaceutically acceptable salt is a salt of an organic acid or a salt of an inorganic acid of the benzimidazole compound.

Preferably, the salt of an organic acid is selected from any one of tartrate, stearate, oxalate, citrate, lactate, sorbate, fumarate, formate, acetate, benzoate, benzenesulfonate, ethanesulfonate, resinate, trifluoroacetate, maleate, malate, L-malate, methanesulfonate, fumarate, an amino acid salt or nicotinate.

Preferably, the salt of an organic acid is selected from any one of tartrate, acetate, maleate, malate, L-malate or fumarate.

Preferably, the salt of an inorganic acid is selected from any one of phosphate, sulfate, nitrate, iodate, bromate, hydroiodide, hydrobromide or hydrochloride.

Preferably, the salt of an inorganic acid is selected from any one of phosphate, sulfate or hydrochloride.

In a fourth aspect, the present application provides a solvate of the benzimidazole compound described above.

Preferably, the solvate is a hydrate and/or an alcoholate of the benzimidazole compound. In the present application, the solvate of the benzimidazole compound has comparable effects as the benzimidazole compound.

In a fifth aspect, the present application provides a prodrug of the benzimidazole compound described above.

In the present application, the prodrug refers to a compound, obtained by modifying a drug in chemical structure, which is inactive or less active *in vitro* and releases an active drug through an enzymatic or non-enzymatic conversion *in vivo* to take effect.

In the present application, the prodrug of the benzimidazole compound is inactive or less active *in vitro* and releases the benzimidazole compound with activity after undergoing metabolic changes *in vivo,* thereby taking effect.

In a sixth aspect, the present application provides a tautomer or a stereochemical isomer of the benzimidazole compound described above. Common tautomers include, but are not limited to, tautomers obtained after the tautomerism of double bonds in benzimidazole rings in the structures of Formula I and Formula II.

In a seventh aspect, the present application provides a pharmaceutical composition. The pharmaceutical composition includes the benzimidazole compound described above.

Preferably, the pharmaceutical composition further includes a pharmaceutically acceptable adjuvant.

Preferably, the pharmaceutically acceptable adjuvant is any one of an excipient, a diluent, a carrier, a flavoring agent, a binder or a filler.

Preferably, the pharmaceutical composition is in a dosage form of an oral preparation, a parenteral preparation or an external-application preparation.

For example, in the present application, the pharmaceutical composition may be prepared into solid, semi-solid, liquid or gas preparations, such as tablets, pills, capsules, powders, granules, ointments, emulsions, suspensions, suppositories, injections, inhalations, gels, microspheres and aerosols.

A typical routine for administering the compound, the pharmaceutically acceptable salt thereof or the pharmaceutical composition thereof in the present application includes, but is not limited to, oral, rectal, topical, inhalation, parenteral, sublingual, intravaginal, intranasal, intraocular, intraperitoneal, intramuscular, subcutaneous or intravenous administration.

In an eighth aspect, the present application provides use of the benzimidazole compound described above and the pharmaceutically acceptable salt of the benzimidazole compound, the solvate, the prodrug, the tautomer or the stereochemical isomer or the pharmaceutical composition for preparing a drug for treating chronic heart failure.

In a ninth aspect, the present application provides use of a substituted benzimidazole compound, a tautomer of the substituted benzimidazole compound, a pharmaceutically acceptable salt of the substituted benzimidazole compound, a solvate of the substituted benzimidazole compound, a hydrate of the substituted benzimidazole compound or a pharmaceutical composition of the substituted benzimidazole compound for preparing a drug for treating chronic heart failure. The substituted benzimidazole compound has the following structure:

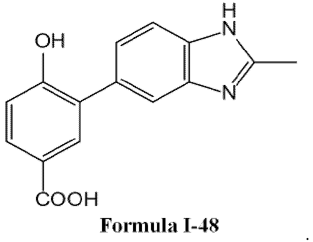

Compared with the existing art, the present application has the beneficial effects below.

The benzimidazole compound and the pharmaceutically acceptable salt of the benzimidazole compound, the solvate, the prodrug, the tautomer or the stereochemical isomer or the pharmaceutical composition of the present application have an effect of treating chronic heart failure and have a broad application prospect.

### BRIEF DESCRIPTION OF DRAWINGS

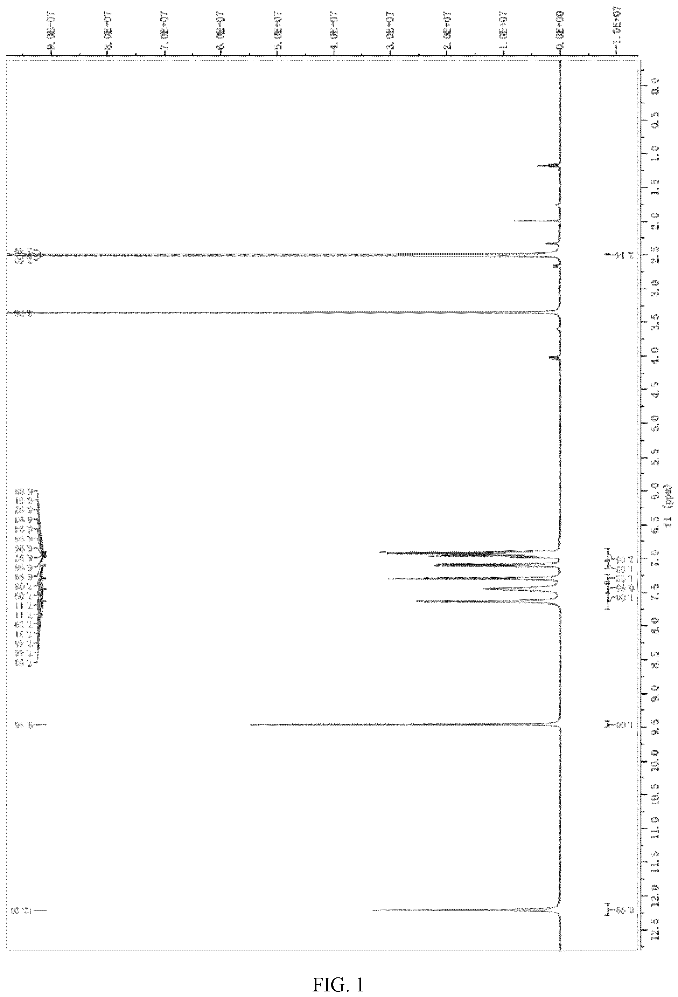

FIG. 1 is a proton nuclear magnetic resonance spectrum of a compound represented by Formula I-1 in the present application.

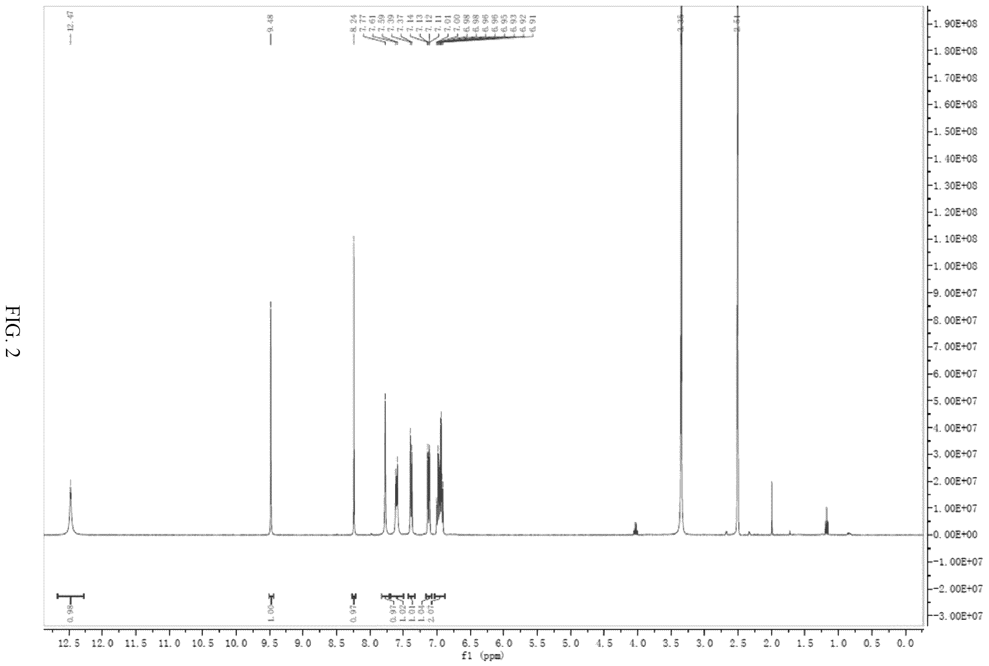

FIG. 2 is a proton nuclear magnetic resonance spectrum of a compound represented by Formula I-24 in the present application.

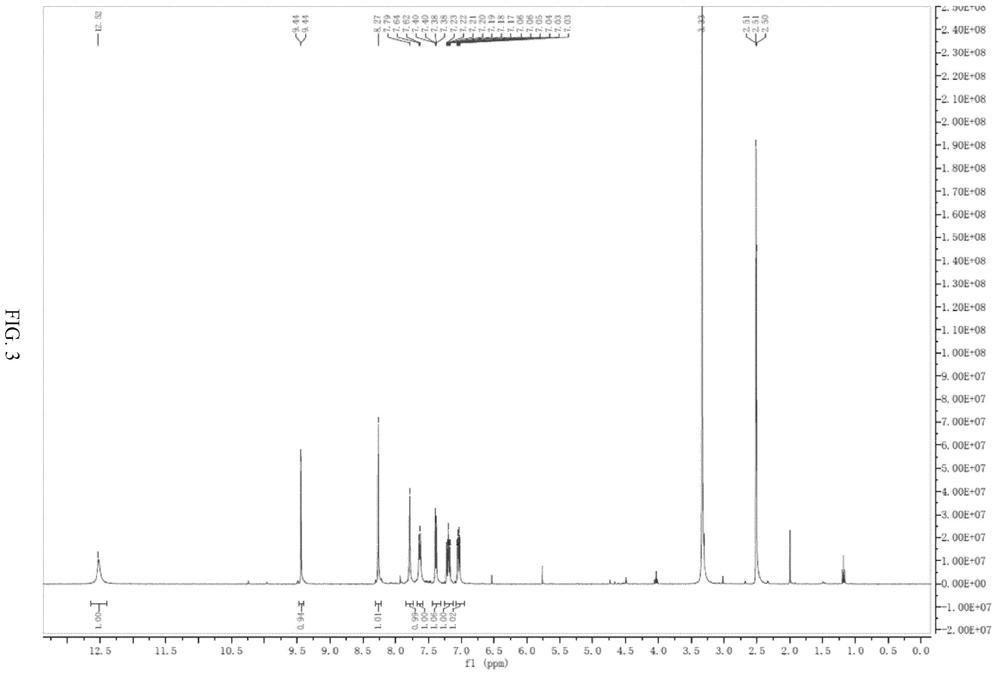

FIG. 3 is a proton nuclear magnetic resonance spectrum of a compound represented by Formula I-25 in the present application.

### DETAILED DESCRIPTION

Technical solutions of the present application are further described below through specific examples. Those skilled in the art are to understand that the examples described herein are used for a better understanding of the present application and are not to be construed as specific limitations to the present application.

### Example 1 Synthesis of Compound Represented by Formula I-1

Under the protection of nitrogen, 2-bromo-4-fluorophenol (19.1 g, 0.1 mol) and (2-methyl-1H-benzimidazol-5-yl)boronic acid (17.6 g, 0.1 mol) were added to 1,4-dioxane (100 ml), and potassium acetate (19.6 g, 0.2 mol), [1,1-bis(diphenylphosphino)ferrocene]palladium(II) dichloride (3.6 g, 5 mmol) and 1,1'-bis(diphenylphosphino)ferrocene (2.8 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-fluoro-2-(2-methyl-1H-benzimidazol-5-yl)phenol as an almost white powdery solid of 13.4 g with a yield of 55.3%. EI-MS M/Z 243.3[M⁺].

¹H-NMR (DMSO-d₆, 400 MHz): 2.49 (3H, s), 6.86-7.02 (2H, m), 7.10 (1H, dd, *J* = 9.7, 2.4 Hz), 7.30 (1H, d, *J* = 8.3 Hz), 7.45 (1H, d, *J* = 6.3 Hz), 7.63 (1H, s), 9.46 (1H, s), 12.20 (1H, s). The proton nuclear magnetic resonance spectrum is shown in FIG .1.

Note: commercialized 5-bromo-1H-benzimidazole was subjected to conventional Miyaura borylation reaction to obtain (2-methyl-1H-benzimidazol-5-yl)boronic acid, and aryl boronic acids in other examples were obtained through the same preparation method or principle; generally, other starting materials may be directly obtained through commercialization, or those skilled in the art may use commercially available raw materials through conventional chemical synthesis methods to obtain other starting materials.

### Example 2 Synthesis of Compound Represented by Formula I-2

Under the protection of nitrogen, 2-bromo-4,6-difluorophenol (2.08 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2,4-difluoro-6-(2-methyl-1H-benzimidazol-5-yl)phenol as an almost white powdery solid of 1.54 g with a yield of 59.2%. EI-MS M/Z 261.2[M⁺].

### Example 3 Synthesis of Compound Represented by Formula I-3

Under the protection of nitrogen, 3-bromo-5-fluoro-2-hydroxybenzonitrile (2.16 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-hydroxy-3-(2-methyl-1H-benzimidazol-5-yl)benzonitrile as an almost white powdery solid of 1.18 g with a yield of 44.2%. EI-MS M/Z 268.3[M⁺].

### Example 4 Synthesis of Compound Represented by Formula I-4

Under the protection of nitrogen, 3-bromo-4-hydroxybenzamide (2.16 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-hydroxy-3-(2-methyl-1H-benzimidazol-5-yl)benzamide as an almost white powdery solid of 0.88 g with a yield of 32.9%. EI-MS M/Z 268.3[M⁺].

### Example 5 Synthesis of Compound Represented by Formula I-5

Under the protection of nitrogen, 3-bromo-5-fluoro-4-hydroxybenzamide (2.34 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-hydroxy-5-(2-methyl-1H-benzimidazol-5-yl)benzamide as an almost white powdery solid of 0.89 g with a yield of 31.2%. EI-MS M/Z 286.3[M⁺].

### Example 6 Synthesis of Compound Represented by Formula I-6

Under the protection of nitrogen, 3-bromo-5-cyano-4-hydroxybenzamide (2.41 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-hydroxy-5-(2-methyl-1H-benzimidazol-5-yl)benzamide as an almost white powdery solid of 0.81 g with a yield of 27.6%. EI-MS M/Z 293.3[M⁺].

### Example 7 Synthesis of Compound Represented by Formula I-7

Under the protection of nitrogen, 3-bromo-4-hydroxy-N,N-dimethylbenzamide (2.16 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-hydroxy-N,N-dimethyl-3-(2-methyl-1H-benzimidazol-5-yl)benzamide as an almost white powdery solid of 1.18 g with a yield of 39.9%. EI-MS M/Z 296.3[M⁺].

### Example 8 Synthesis of Compound Represented by Formula I-8

Under the protection of nitrogen,
3-bromo-5-fluoro-4-hydroxy-N,N-dimethylbenzamide (2.60 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-hydroxy-N,N-dimethyl-5-(2-methyl-1H-benzimidazol-5-yl)benzamide as an almost white powdery solid of 1.19 g with a yield of 38.0%. EI-MS M/Z 314.3[M⁺].

### Example 9 Synthesis of Compound Represented by Formula I-9

Under the protection of nitrogen,
3-bromo-5-cyano-4-hydroxy-N,N-dimethylbenzamide (2.69 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-hydroxy-N,N-dimethyl-5-(2-methyl-1H-benzimidazol-5-yl)benzamide as an almost white powdery solid of 1.15 g with a yield of 35.9%. EI-MS M/Z 321.4[M⁺].

### Example 10 Synthesis of Compound Represented by Formula I-48

Under the protection of nitrogen, 3-bromo-4-hydroxybenzoic acid (2.17 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-hydroxy-3-(2-methyl-1H-benzimidazol-5-yl)benzoic acid as an almost white powdery solid of 0.78 g with a yield of 26.1%. EI-MS M/Z 269.3[M⁺].

### Example 11 Synthesis of Compound Represented by Formula I-10

Under the protection of nitrogen, 3-bromo-5-fluoro-4-hydroxybenzoic acid (2.35 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-hydroxy-5-(2-methyl-1H-benzimidazol-5-yl)benzoic acid as an almost white powdery solid of 0.69 g with a yield of 24.1%. EI-MS M/Z 287.3[M⁺].

### Example 12 Synthesis of Compound Represented by Formula I-11

Under the protection of nitrogen, 3-bromo-5-cyano-4-hydroxybenzoic acid (2.42 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-hydroxy-5-(2-methyl-1H-benzimidazol-5-yl)benzoic acid as an almost white powdery solid of 0.61 g with a yield of 20.8%. EI-MS M/Z 294.3[M⁺].

### Example 13 Synthesis of Compound Represented by Formula I-12

Under the protection of nitrogen, methyl 3-bromo-4-hydroxy benzoate (2.31 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-hydroxy-3-(2-methyl-1H-benzimidazol-5-yl) benzoate as an almost white powdery solid of 1.58 g with a yield of 56.0%. EI-MS M/Z 283.3[M⁺].

### Example 14 Synthesis of Compound Represented by Formula I-13

Under the protection of nitrogen, methyl 3-bromo-5-fluoro-4-hydroxy benzoate (2.49 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-hydroxy-5-(2-methyl-1H-benzimidazol-5-yl) benzoate as an almost white powdery solid of 1.69 g with a yield of 56.3%. EI-MS M/Z 301.3[M⁺].

### Example 15 Synthesis of Compound Represented by Formula I-14

Under the protection of nitrogen, methyl 3-bromo-5-cyano-4-hydroxy benzoate (2.56 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-hydroxy-5-(2-methyl-1H-benzimidazol-5-yl) benzoate as an almost white powdery solid of 1.61 g with a yield of 43.2%. EI-MS M/Z 308.3[M⁺].

### Example 16 Synthesis of Compound Represented by Formula I-15

Under the protection of nitrogen, 4-allyl-2-bromophenol (2.13 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-allyl-2-(2-methyl-1H-benzimidazol-5-yl)phenol as an almost white powdery solid of 0.78 g with a yield of 29.4%. EI-MS M/Z 265.3[M⁺].

### Example 17 Synthesis of Compound Represented by Formula I-16

Under the protection of nitrogen, 4-allyl-2-bromo-6-fluorophenol (2.31 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-allyl-2-(2-methyl-1H-benzimidazol-5-yl)-6-fluorophenol as an almost white powdery solid of 0.69 g with a yield of 24.4%. EI-MS M/Z 283.3[M⁺].

### Example 18 Synthesis of Compound Represented by Formula I-17

Under the protection of nitrogen, 5-allyl-3-bromo-2-hydroxybenzonitrile (2.38 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-3-(2-methyl-1H-benzimidazol-5-yl)-2-hydroxybenzonitrile as an almost white powdery solid of 0.81 g with a yield of 28.0%. EI-MS M/Z 290.3[M⁺].

### Example 19 Synthesis of Compound Represented by Formula I-18

Under the protection of nitrogen, 2-(3-bromo-4-hydroxyphenyl)acetonitrile (2.12 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-hydroxy-3-(2-methyl-1H-benzimidazol-5-yl)phenyl)acetonitrile as an almost white powdery solid of 1.28 g with a yield of 48.7%. EI-MS M/Z 264.3[M⁺].

### Example 20 Synthesis of Compound Represented by Formula I-19

Under the protection of nitrogen, 2-(3-bromo-5-fluoro-4-phenol)acetonitrile (2.30 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-hydroxy-5-(2-methyl-1H-benzimidazol-5-yl)phenyl)acetonitrile as an almost white powdery solid of 1.19 g with a yield of 42.3%. EI-MS M/Z 282.3[M⁺].

### Example 21 Synthesis of Compound Represented by Formula I-20

Under the protection of nitrogen, 3-bromo-5-cyanomethyl-2-hydroxybenzonitrile (2.37 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-hydroxy-3-(2-methyl-1H-benzimidazol-5-yl)benzonitrile as an almost white powdery solid of 1.01 g with a yield of 35.0%. EI-MS M/Z 289.3[M⁺].

### Example 22 Synthesis of Compound Represented by Formula I-21

Under the protection of nitrogen, 2-bromo-4-propylphenol (2.15 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(2-methyl-1H-benzimidazol-5-yl)-4-propylphenol as an almost white powdery solid of 1.75 g with a yield of 65.5%. EI-MS M/Z 267.3[M⁺].

### Example 23 Synthesis of Compound Represented by Formula I-22

Under the protection of nitrogen, 2-bromo-6-fluoro-4-propylphenol (2.33 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-fluoro-6-(2-methyl-1H-benzimidazol-5-yl)-4-propylphenol as an almost white powdery solid of 1.39 g with a yield of 48.7%. EI-MS M/Z 285.3[M⁺].

### Example 24 Synthesis of Compound Represented by Formula I-23

Under the protection of nitrogen, 3-bromo-2-hydroxy-2-propylbenzonitrile (2.40 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-hydroxy-3-(2-methyl-1H-benzimidazol-5-yl)-5-propylbenzonitrile as an almost white powdery solid of 1.11 g with a yield of 38.0%. EI-MS M/Z 292.4[M⁺].

### Example 25 Synthesis of Compound Represented by Formula I-24

Under the protection of nitrogen, 2-bromo-4-fluorophenol (19.1 g, 0.1 mol) and (1H-benzimidazol-5-yl)boronic acid (16.2 g, 0.1 mol) were added to 1,4-dioxane (100 ml), and potassium acetate (19.6 g, 0.2 mol), [1,1-bis(diphenylphosphino)ferrocene]palladium(II) dichloride (3.6 g, 5 mmol) and 1,1'-bis(diphenylphosphino)ferrocene (2.8 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-fluoro-2-(1H-benzimidazol-5-yl)phenol as an almost white powdery solid of 14.5 g with a yield of 63.5%. EI-MS M/Z 229.2[M⁺].

¹H-NMR (DMSO-d₆, 400 MHz): 6.91-7.01 (2H, m), 7.12 (1H, dd, J = 9.8, 3.0 Hz), 7.38 (1H, d, J = 8.4 Hz), 7.60 (1H, d, J = 8.3 Hz), 7.77 (1H, s), 8.24 (1H, s), 9.48 (1H, s), 12.47 (1H, s). The proton nuclear magnetic resonance spectrum is shown in FIG .2.

### Example 26 Synthesis of Compound Represented by Formula I-25

Under the protection of nitrogen, 2-bromo-4,6-difluorophenol (2.08 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2,4-difluoro-6-(1H-benzimidazol-5-yl)phenol as an almost white powdery solid of 1.57 g with a yield of 63.8%. EI-MS M/Z 247.2[M⁺].

¹H-NMR (DMSO-d₆, 400 MHz): 7.03-7.06 (1H, m), 7.17-7.23 (1H, m), 7.39 (1H, dd, J = 8.4 Hz), 7.63 (1H, d, J = 8.4 Hz), 7.79 (1H, s), 8.27 (1H, s), 9.44 (1H, s), 12.52 (1H, s). The proton nuclear magnetic resonance spectrum is shown in FIG .3.

### Example 27 Synthesis of Compound Represented by Formula 1-26

Under the protection of nitrogen, 3-bromo-5-fluoro-2-hydroxybenzonitrile (2.16 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-hydroxy-3-(1H-benzimidazol-5-yl)benzonitrile as an almost white powdery solid of 1.12 g with a yield of 44.3%. EI-MS M/Z 254.2[M⁺].

### Example 28 Synthesis of Compound Represented by Formula I-27

Under the protection of nitrogen, 3-bromo-4-hydroxybenzamide (2.16 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-hydroxy-3-(1H-benzimidazol-5-yl)benzamide as an almost white powdery solid of 0.86 g with a yield of 34.0%. EI-MS M/Z 254.3[M⁺].

### Example 29 Synthesis of Compound Represented by Formula I-28

Under the protection of nitrogen, 3-bromo-5-fluoro-4-hydroxybenzamide (2.34 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-hydroxy-5-(1H-benzimidazol-5-yl)benzamide as an almost white powdery solid of 0.82 g with a yield of 30.1%. EI-MS M/Z 272.3[M⁺].

### Example 30 Synthesis of Compound Represented by Formula I-29

Under the protection of nitrogen, 3-bromo-5-cyano-4-hydroxybenzamide (2.41 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-hydroxy-5-(1H-benzimidazol-5-yl)benzamide as an almost white powdery solid of 0.80 g with a yield of 26.9%. EI-MS M/Z 279.3[M⁺].

### Example 31 Synthesis of Compound Represented by Formula I-30

Under the protection of nitrogen, 3-bromo-4-hydroxy-N,N-dimethylbenzamide (2.16 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-hydroxy-N,N-dimethyl-3-(1H-benzimidazol-5-yl)benzamide as an almost white powdery solid of 1.12 g with a yield of 39.9%. EI-MS M/Z 282.3[M⁺].

### Example 32 Synthesis of Compound Represented by Formula I-31

Under the protection of nitrogen,
3-bromo-5-fluoro-4-hydroxy-N,N-dimethylbenzamide (2.60 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-hydroxy-N,N-dimethyl-5-(1H-benzimidazol-5-yl)benzamide as an almost white powdery solid of 1.09 g with a yield of 36.4%. EI-MS M/Z 300.3[M⁺].

### Example 33 Synthesis of Compound Represented by Formula I-32

Under the protection of nitrogen,
3-bromo-5-cyano-4-hydroxy-N,N-dimethylbenzamide (2.69 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-hydroxy-N,N-dimethyl-5-(1H-benzimidazol-5-yl)benzamide as an almost white powdery solid of 1.05 g with a yield of 34.3%. EI-MS M/Z 307.3[M⁺].

### Example 34 Synthesis of Compound Represented by Formula I-33

Under the protection of nitrogen, 3-bromo-4-hydroxybenzoic acid (2.17 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-hydroxy-3-(1H-benzimidazol-5-yl)benzoic acid as an almost white powdery solid of 0.68 g with a yield of 26.8%. EI-MS M/Z 255.3[M⁺].

### Example 35 Synthesis of Compound Represented by Formula I-34

Under the protection of nitrogen, 3-bromo-5-fluoro-4-hydroxybenzoic acid (2.35 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-hydroxy-5-(1H-benzimidazol-5-yl)benzoic acid as an almost white powdery solid of 0.52 g with a yield of 19.0%. EI-MS M/Z 273.2[M⁺].

### Example 36 Synthesis of Compound Represented by Formula I-35

Under the protection of nitrogen, 3-bromo-5-cyano-4-hydroxybenzoic acid (2.42 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-hydroxy-5-(1H-benzimidazol-5-yl)benzoic acid as an almost white powdery solid of 0.63 g with a yield of 22.5%. EI-MS M/Z 280.3[M⁺].

### Example 37 Synthesis of Compound Represented by Formula I-36

Under the protection of nitrogen, methyl 3-bromo-4-hydroxy benzoate (2.31 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-hydroxy-3-(1H-benzimidazol-5-yl) benzoate as an almost white powdery solid of 1.50 g with a yield of 56.0%. EI-MS M/Z 269.3[M⁺].

### Example 38 Synthesis of Compound Represented by Formula I-37

Under the protection of nitrogen, methyl 3-bromo-5-fluoro-4-hydroxy benzoate (2.49 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-hydroxy-5-(1H-benzimidazol-5-yl) benzoate as an almost white powdery solid of 1.62 g with a yield of 56.6%. EI-MS M/Z 287.3[M⁺].

### Example 39 Synthesis of Compound Represented by Formula I-38

Under the protection of nitrogen, methyl 3-bromo-5-cyano-4-hydroxy benzoate (2.56 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-hydroxy-5-(1H-benzimidazol-5-yl) benzoate as an almost white powdery solid of 1.60 g with a yield of 54.6%. EI-MS M/Z 294.3[M⁺].

### Example 40 Synthesis of Compound Represented by Formula I-39

Under the protection of nitrogen, 4-allyl-2-bromophenol (2.13 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-allyl-2-(1H-benzimidazol-5-yl)phenol as an almost white powdery solid of 0.68 g with a yield of 27.2%. EI-MS M/Z 251.3[M⁺].

### Example 41 Synthesis of Compound Represented by Formula I-40

Under the protection of nitrogen, 4-allyl-2-bromo-6-fluorophenol (2.31 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-allyl-2-(1H-benzimidazol-5-yl)-6-fluorophenol as an almost white powdery solid of 0.65 g with a yield of 24.2%. EI-MS M/Z 269.3[M⁺].

### Example 42 Synthesis of Compound Represented by Formula I-41

Under the protection of nitrogen, 5-allyl-3-bromo-2-hydroxybenzonitrile (2.38 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-3-(1H-benzimidazol-5-yl)-2-hydroxybenzonitrile as an almost white powdery solid of 0.80 g with a yield of 29.1%. EI-MS M/Z 276.3[M⁺].

### Example 43 Synthesis of Compound Represented by Formula I-42

Under the protection of nitrogen, 2-(3-bromo-4-hydroxyphenyl)acetonitrile (2.12 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-hydroxy-3-(1H-benzimidazol-5-yl)phenyl)acetonitrile as an almost white powdery solid of 1.28 g with a yield of 51.2%. EI-MS M/Z 250.3[M⁺].

### Example 44 Synthesis of Compound Represented by Formula I-43

Under the protection of nitrogen, 2-(3-bromo-5-fluoro-4-phenol)acetonitrile (2.30 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-hydroxy-5-(1H-benzimidazol-5-yl)phenyl)acetonitrile as an almost white powdery solid of 1.12 g with a yield of 41.9%. EI-MS M/Z 268.3[M⁺].

### Example 45 Synthesis of Compound Represented by Formula I-44

Under the protection of nitrogen, 3-bromo-5-cyanomethyl-2-hydroxybenzonitrile (2.37 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-hydroxy-3-(1H-benzimidazol-5-yl)benzonitrile as an almost white powdery solid of 1.12 g with a yield of 40.9%. EI-MS M/Z 275.3[M⁺].

### Example 46 Synthesis of Compound Represented by Formula I-45

Under the protection of nitrogen, 2-bromo-4-propylphenol (2.15 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(1H-benzimidazol-5-yl)-4-propylphenol as an almost white powdery solid of 1.55 g with a yield of 61.5%. EI-MS M/Z 253.3[M⁺].

### Example 47 Synthesis of Compound Represented by Formula I-46

Under the protection of nitrogen, 2-bromo-6-fluoro-4-propylphenol (2.33 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-fluoro-6-(1H-benzimidazol-5-yl)-4-propylphenol as an almost white powdery solid of 1.33 g with a yield of 49.2%. EI-MS M/Z 271.3[M⁺].

### Example 48 Synthesis of Compound Represented by Formula I-47

Under the protection of nitrogen, 3-bromo-2-hydroxy-5-propylbenzonitrile (2.40 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-hydroxy-3-(1H-benzimidazol-5-yl)-5-propylbenzonitrile as an almost white powdery solid of 1.02 g with a yield of 36.8%. EI-MS M/Z 278.3[M⁺].

### Example 49 Synthesis of Compound Represented by Formula II-1

Under the protection of nitrogen, 2-(2-bromo-4-fluorophenyl)propane-2-ol (2.31 g, 0.1 mol) and (2-methyl-1H-benzimidazol-5-yl)boronic acid (1.76 g, 0.1 mol) were added to 1,4-dioxane (10 ml), and potassium acetate (1.96 g, 0.2 mol), [1,1-bis(diphenylphosphino)ferrocene]palladium(II) dichloride (0.36 g, 5 mmol) and 1,1'-bis(diphenylphosphino)ferrocene (0.28 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-fluoro-2-(2-methyl-1H-benzimidazol-5-yl)phenyl)propane-2-ol as an almost white powdery solid of 1.24 g with a yield of 43.7%. EI-MS M/Z 285.3[M⁺].

### Example 50 Synthesis of Compound Represented by Formula II-2

Under the protection of nitrogen, 2-(2-bromo-4,6-difluorophenyl)propane-2-ol (2.50 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(2,4-difluoro-6-(2-methyl-1H-benzimidazol-5-yl)phenyl)propane-2-ol as an almost white powdery solid of 1.59 g with a yield of 52.5%. EI-MS M/Z 303.3[M⁺].

### Example 51 Synthesis of Compound Represented by Formula II-3

Under the protection of nitrogen,
3-bromo-5-fluoro-2-(2-hydroxypropan-2-yl)benzonitrile (2.58 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-(2-hydroxypropan-2-yl)-3-(2-methyl-1H-benzimidazol-5-yl)benzonitrile as an almost white powdery solid of 1.28 g with a yield of 41.4%. EI-MS M/Z 310.3[M⁺].

### Example 52 Synthesis of Compound Represented by Formula II-4

Under the protection of nitrogen, 3-bromo-4-(2-hydroxypropan-2-yl)benzamide (2.58 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(2-hydroxypropan-2-yl)-3-(2-methyl-1H-benzimidazol-5-yl)benzamide as an almost white powdery solid of 0.78 g with a yield of 25.4%. EI-MS M/Z 310.4[M⁺].

### Example 53 Synthesis of Compound Represented by Formula II-5

Under the protection of nitrogen,
3-bromo-5-fluoro-4-(2-hydroxypropan-2-yl)benzamide (2.76 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(2-hydroxypropan-2-yl)-5-(2-methyl-1H-benzimidazol-5-yl)benzamide as an almost white powdery solid of 0.89 g with a yield of 27.1%. EI-MS M/Z 328.4[M⁺].

### Example 54 Synthesis of Compound Represented by Formula II-6

Under the protection of nitrogen,
3-bromo-5-cyano-4-(2-hydroxypropan-2-yl)benzamide (2.83 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(2-hydroxypropan-2-yl)-5-(2-methyl-1H-benzimidazol-5-yl)benzamide as an almost white powdery solid of 0.71 g with a yield of 21.2%. EI-MS M/Z 335.4[M⁺].

### Example 55 Synthesis of Compound Represented by Formula II-7

Under the protection of nitrogen,
3-bromo-4-(2-hydroxypropan-2-yl)-N,N-dimethylbenzamide (2.86 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(2-hydroxypropan-2-yl)-N,N-dimethyl-3-(2-methyl-1H-benzimidazol-5-yl)benzamide as an almost white powdery solid of 1.18 g with a yield of 35.0%. EI-MS M/Z 338.4[M⁺].

### Example 56 Synthesis of Compound Represented by Formula II-8

Under the protection of nitrogen,
3-bromo-5-fluoro-4-(2-hydroxypropan-2-yl)-N,N-dimethylbenzamide (3.04 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(2-hydroxypropan-2-yl)-N,N-dimethyl-5-(2-methyl-1H-benzimidazol-5-yl)benzamid e as an almost white powdery solid of 1.21 g with a yield of 34.0%. EI-MS M/Z 356.4[M⁺].

### Example 57 Synthesis of Compound Represented by Formula II-9

Under the protection of nitrogen,
3-bromo-5-cyano-4-(2-hydroxypropan-2-yl)-N,N-dimethylbenzamide (3.11 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(2-hydroxypropan-2-yl)-N,N-dimethyl-5-(2-methyl-1H-benzimidazol-5-yl)benzamid e as an almost white powdery solid of 1.20 g with a yield of 33.1%. EI-MS M/Z 363.4[M⁺].

### Example 58 Synthesis of Compound Represented by Formula II-10

Under the protection of nitrogen, 3-bromo-4-(2-hydroxypropan-2-yl)benzoic acid (2.59 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(2-hydroxypropan-2-yl)-3-(2-methyl-1H-benzimidazol-5-yl)benzoic acid as an almost white powdery solid of 0.79 g with a yield of 25.4%. EI-MS M/Z 311.4[M⁺].

### Example 59 Synthesis of Compound Represented by Formula II-11

Under the protection of nitrogen, 3-bromo-5-fluoro-4-(2-hydroxypropan-2-yl)benzoic acid (2.77 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(2-hydroxypropan-2-yl)-5-(2-methyl-1H-benzimidazol-5-yl)benzoic acid as an almost white powdery solid of 0.74 g with a yield of 22.5%. EI-MS M/Z 329.3[M⁺].

### Example 60 Synthesis of Compound Represented by Formula 11-12

Under the protection of nitrogen, 3-bromo-5-cyano-4-(2-hydroxypropan-2-yl)benzoic acid (2.84 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(2-hydroxypropan-2-yl)-5-(2-methyl-1H-benzimidazol-5-yl)benzoic acid as an almost white powdery solid of 0.63 g with a yield of 19.4%. EI-MS M/Z 336.4[M⁺].

### Example 61 Synthesis of Compound Represented by Formula 11-13

Under the protection of nitrogen, methyl 3-bromo-4-(2-hydroxypropan-2-yl) benzoate (2.73 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-(2-hydroxypropan-2-yl)-3-(2-methyl-1H-benzimidazol-5-yl) benzoate as an almost white powdery solid of 1.55 g with a yield of 47.8%. EI-MS M/Z 325.4[M⁺].

### Example 62 Synthesis of Compound Represented by Formula 11-14

Under the protection of nitrogen, methyl 3-bromo-5-fluoro-4-(2-hydroxypropan-2-yl) benzoate (2.91 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-(2-hydroxypropan-2-yl)-5-(2-methyl-1H-benzimidazol-5-yl) benzoate as an almost white powdery solid of 1.69 g with a yield of 49.4%. EI-MS M/Z 343.4[M⁺].

### Example 63 Synthesis of Compound Represented by Formula II-15

Under the protection of nitrogen, methyl 3-bromo-5-cyano-4-(2-hydroxypropan-2-yl) benzoate (2.98 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-(2-hydroxypropan-2-yl)-5-(2-methyl-1H-benzimidazol-5-yl) benzoate as an almost white powdery solid of 1.63 g with a yield of 46.5%. EI-MS M/Z 350.4[M⁺].

### Example 64 Synthesis of Compound Represented by Formula 11-16

Under the protection of nitrogen, 2-(4-allyl-2-bromophenyl)propane-2-ol (2.55 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-allyl-2-(2-methyl-1H-benzimidazol-5-yl)phenyl)propane-2-ol as an almost white powdery solid of 0.68 g with a yield of 22.2%. EI-MS M/Z 307.4[M⁺].

### Example 65 Synthesis of Compound Represented by Formula II-17

Under the protection of nitrogen, 2-(4-allyl-2-bromo-6-fluorophenyl)propane-2-ol (2.73 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-allyl-2-fluoro-6-(2-methyl-1H-benzimidazol-5-yl)phenyl)propane-2-ol as an almost white powdery solid of 0.59 g with a yield of 18.2%. EI-MS M/Z 325.4[M⁺].

### Example 66 Synthesis of Compound Represented by Formula II-18

Under the protection of nitrogen,
5-allyl-3-bromo-2-(2-hydroxypropan-2-yl)benzonitrile (2.80 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-2-(2-hydroxypropan-2-yl)-3-(2-methyl-1H-benzimidazol-5-yl)benzonitrile as an almost white powdery solid of 0.61 g with a yield of 18.4%. EI-MS M/Z 332.4[M⁺].

### Example 67 Synthesis of Compound Represented by Formula 11-19

Under the protection of nitrogen,
2-(3-bromo-4-(2-hydroxypropan-2-yl)phenyl)acetonitrile (2.54 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-(2-hydroxypropan-2-yl)-3-(2-methyl-1H-benzimidazol-5-yl)phenyl)acetonitrile as an almost white powdery solid of 1.08 g with a yield of 35.4%. EI-MS M/Z 306.4[M⁺].

### Example 68 Synthesis of Compound Represented by Formula II-20

Under the protection of nitrogen,
2-(3-bromo-5-fluoro-4-(2-hydroxypropan-2-yl)phenyl)acetonitrile (2.72 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-(2-hydroxypropan-2-yl)-5-(2-methyl-1H-benzimidazol-5-yl)phenyl)acetonitrile as an almost white powdery solid of 1.11 g with a yield of 34.3%. EI-MS M/Z 324.4[M⁺].

### Example 69 Synthesis of Compound Represented by Formula II-21

Under the protection of nitrogen,
3-bromo-5-cyanomethyl-2-(2-hydroxypropan-2-yl)benzonitrile (2.79 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-(2-hydroxypropan-2-yl)-3-(2-methyl-1H-benzimidazol-5-yl)benzonitrile as an almost white powdery solid of 0.93 g with a yield of 28.1%. EI-MS M/Z 331.4[M⁺].

### Example 70 Synthesis of Compound Represented by Formula II-22

Under the protection of nitrogen, 2-(2-bromo-4-propylphenyl)propane-2-ol (2.57 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(2-(2-methyl-1H-benzimidazol-5-yl)-4-propylphenyl)propane-2-ol as an almost white powdery solid of 1.65 g with a yield of 53.5%. EI-MS M/Z 309.4[M⁺].

### Example 71 Synthesis of Compound Represented by Formula II-23

Under the protection of nitrogen, 2-(2-bromo-6-fluoro-4-propylphenyl)propane-2-ol (2.75 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(2-fluoro-6-(2-methyl-1H-benzimidazol-5-yl)-4-propylphenyl)propane-2-ol as an almost white powdery solid of 1.33 g with a yield of 40.7%. EI-MS M/Z 327.4[M⁺].

### Example 72 Synthesis of Compound Represented by Formula II-24

Under the protection of nitrogen,
3-bromo-2-(2-hydroxypropan-2-yl)-5-propanebenzonitrile (2.82 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-hydroxy-3-(2-methyl-1H-benzimidazol-5-yl)-5-propylbenzonitrile as an almost white powdery solid of 1.01 g with a yield of 30.3%. EI-MS M/Z 334.4[M⁺].

### Example 73 Synthesis of Compound Represented by Formula II-25

Under the protection of nitrogen, 2-(2-bromo-4-fluorophenyl)propane-2-ol (2.31 g, 0.1 mol) and (1H-benzimidazol-5-yl)boronic acid (1.62 g, 0.1 mol) were added to 1,4-dioxane (10 ml), and potassium acetate (1.96 g, 0.2 mol), [1,1-bis(diphenylphosphino)ferrocene]palladium(II) dichloride (0.36 g, 5 mmol) and 1,1'-bis(diphenylphosphino)ferrocene (0.28 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-fluoro-2-(1H-benzimidazol-5-yl)phenyl)propane-2-ol as an almost white powdery solid of 1.21 g with a yield of 44.8%. EI-MS M/Z 271.3[M⁺].

### Example 74 Synthesis of Compound Represented by Formula II-26

Under the protection of nitrogen, 2-(2-bromo-4,6-difluorophenyl)propane-2-ol (2.50 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(2,4-difluoro-6-(1H-benzimidazol-5-yl)phenyl)propane-2-ol as an almost white powdery solid of 1.44 g with a yield of 49.9%. EI-MS M/Z 289.3[M⁺].

### Example 75 Synthesis of Compound Represented by Formula II-27

Under the protection of nitrogen,
3-bromo-5-fluoro-2-(2-hydroxypropan-2-yl)benzonitrile (2.58 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-(2-hydroxypropan-2-yl)-3-(1H-benzimidazol-5-yl)benzonitrile as an almost white powdery solid of 1.08 g with a yield of 36.6%. EI-MS M/Z 296.3[M⁺].

### Example 76 Synthesis of Compound Represented by Formula II-28

Under the protection of nitrogen, 3-bromo-4-(2-hydroxypropan-2-yl)benzamide (2.58 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110°C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(2-hydroxypropan-2-yl)-3-(1H-benzimidazol-5-yl)benzamide as an almost white powdery solid of 0.72 g with a yield of 24.4%. EI-MS M/Z 296.3[M⁺].

### Example 77 Synthesis of Compound Represented by Formula II-29

Under the protection of nitrogen,
3-bromo-5-fluoro-4-(2-hydroxypropan-2-yl)benzamide (2.76 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(2-hydroxypropan-2-yl)-5-(1H-benzimidazol-5-yl)benzamide as an almost white powdery solid of 0.83 g with a yield of 26.5%. EI-MS M/Z 314.3[M⁺].

### Example 78 Synthesis of Compound Represented by Formula II-30

Under the protection of nitrogen,
3-bromo-5-cyano-4-(2-hydroxypropan-2-yl)benzamide (2.83 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(2-hydroxypropan-2-yl)-5-(1H-benzimidazol-5-yl)benzamide as an almost white powdery solid of 0.66 g with a yield of 20.6%. EI-MS M/Z 321.4[M⁺].

### Example 79 Synthesis of Compound Represented by Formula II-31

Under the protection of nitrogen,
3-bromo-4-(2-hydroxypropan-2-yl)-N,N-dimethylbenzamide (2.86 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(2-hydroxypropan-2-yl)-N,N-dimethyl-3-(1H-benzimidazol-5-yl)benzamide as an almost white powdery solid of 1.03 g with a yield of 31.8%. EI-MS M/Z 324.4[M⁺].

### Example 80 Synthesis of Compound Represented by Formula II-32

Under the protection of nitrogen,
3-bromo-5-fluoro-4-(2-hydroxypropan-2-yl)-N,N-dimethylbenzamide (3.04 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(2-hydroxypropan-2-yl)-N,N-dimethyl-5-(1H-benzimidazol-5-yl)benzamide as an almost white powdery solid of 1.04 g with a yield of 30.5%. EI-MS M/Z 342.4[M⁺].

### Example 81 Synthesis of Compound Represented by Formula II-33

Under the protection of nitrogen,
3-bromo-5-cyano-4-(2-hydroxypropan-2-yl)-N,N-dimethylbenzamide (3.11 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(2-hydroxypropan-2-yl)-N,N-dimethyl-5-(1H-benzimidazol-5-yl)benzamide as an almost white powdery solid of 1.09 g with a yield of 31.3%. EI-MS M/Z 349.4[M⁺].

### Example 82 Synthesis of Compound Represented by Formula II-34

Under the protection of nitrogen, 3-bromo-4-(2-hydroxypropan-2-yl)benzoic acid (2.59 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(2-hydroxypropan-2-yl)-3-(1H-benzimidazol-5-yl)benzoic acid as an almost white powdery solid of 0.77 g with a yield of 26.0%. EI-MS M/Z 297.3[M⁺].

### Example 83 Synthesis of Compound Represented by Formula II-35

Under the protection of nitrogen, 3-bromo-5-fluoro-4-(2-hydroxypropan-2-yl)benzoic acid (2.77 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(2-hydroxypropan-2-yl)-5-(1H-benzimidazol-5-yl)benzoic acid as an almost white powdery solid of 0.76 g with a yield of 24.2%. EI-MS M/Z 315.3[M⁺].

### Example 84 Synthesis of Compound Represented by Formula II-36

Under the protection of nitrogen, 3-bromo-5-cyano-4-(2-hydroxypropan-2-yl)benzoic acid (2.84 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(2-hydroxypropan-2-yl)-5-(1H-benzimidazol-5-yl)benzoic acid as an almost white powdery solid of 0.74 g with a yield of 23.0%. EI-MS M/Z 322.3[M⁺].

### Example 85 Synthesis of Compound Represented by Formula II-37

Under the protection of nitrogen, methyl 3-bromo-4-(2-hydroxypropan-2-yl) benzoate (2.73 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-(2-hydroxypropan-2-yl)-3-(1H-benzimidazol-5-yl) benzoate as an almost white powdery solid of 1.25 g with a yield of 40.3%. EI-MS M/Z 311.4[M⁺].

### Example 86 Synthesis of Compound Represented by Formula II-38

Under the protection of nitrogen, methyl 3-bromo-5-fluoro-4-(2-hydroxypropan-2-yl) benzoate (2.91 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-(2-hydroxypropan-2-yl)-5-(1H-benzimidazol-5-yl) benzoate as an almost white powdery solid of 1.89 g with a yield of 57.6%. EI-MS M/Z 329.3[M⁺].

### Example 87 Synthesis of Compound Represented by Formula II-39

Under the protection of nitrogen, methyl 3-bromo-5-cyano-4-(2-hydroxypropan-2-yl) benzoate (2.98 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-(2-hydroxypropan-2-yl)-5-(1H-benzimidazol-5-yl) benzoate as an almost white powdery solid of 1.68 g with a yield of 50.1%. EI-MS M/Z 336.4[M⁺].

### Example 88 Synthesis of Compound Represented by Formula II-40

Under the protection of nitrogen, 2-(4-allyl-2-bromophenyl)propane-2-ol (2.55 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-allyl-2-(1H-benzimidazol-5-yl)phenyl)propane-2-ol as an almost white powdery solid of 0.61 g with a yield of 20.9%. EI-MS M/Z 293.4[M⁺].

### Example 89 Synthesis of Compound Represented by Formula II-41

Under the protection of nitrogen, 2-(4-allyl-2-bromo-6-fluorophenyl)propane-2-ol (2.73 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-allyl-2-fluoro-6-(1H-benzimidazol-5-yl)phenyl)propane-2-ol as an almost white powdery solid of 0.50 g with a yield of 16.1%. EI-MS M/Z 311.4[M⁺].

### Example 90 Synthesis of Compound Represented by Formula II-42

Under the protection of nitrogen,
5-allyl-3-bromo-2-(2-hydroxypropan-2-yl)benzonitrile (2.80 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-2-(2-hydroxypropan-2-yl)-3-(1H-benzimidazol-5-yl)benzonitrile as an almost white powdery solid of 0.56 g with a yield of 17.6%. EI-MS M/Z 318.4[M⁺].

### Example 91 Synthesis of Compound Represented by Formula II-43

Under the protection of nitrogen,
2-(3-bromo-4-(2-hydroxypropan-2-yl)phenyl)acetonitrile (2.54 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-(2-hydroxypropan-2-yl)-3-(1H-benzimidazol-5-yl)phenyl)acetonitrile as an almost white powdery solid of 1.02 g with a yield of 26.1%. EI-MS M/Z 292.4[M⁺].

### Example 92 Synthesis of Compound Represented by Formula II-44

Under the protection of nitrogen,
2-(3-bromo-5-fluoro-4-(2-hydroxypropan-2-yl)phenyl)acetonitrile (2.72 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-(2-hydroxypropan-2-yl)-5-(1H-benzimidazol-5-yl)phenyl)acetonitrile as an almost white powdery solid of 1.31 g with a yield of 42.3%. EI-MS M/Z 310.3[M⁺].

### Example 93 Synthesis of Compound Represented by Formula II-45

Under the protection of nitrogen,
3-bromo-5-cyanomethyl-2-(2-hydroxypropan-2-yl)benzonitrile (2.79 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-(2-hydroxypropan-2-yl)-3-(1H-benzimidazol-5-yl)benzonitrile as an almost white powdery solid of 1.03 g with a yield of 32.6%. EI-MS M/Z 317.4[M⁺].

### Example 94 Synthesis of Compound Represented by Formula II-46

Under the protection of nitrogen, 2-(2-bromo-4-propylphenyl)propane-2-ol (2.57 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(2-(1H-benzimidazol-5-yl)-4-propylphenyl)propane-2-ol as an almost white powdery solid of 1.66 g with a yield of 56.4%. EI-MS M/Z 295.4[M⁺].

### Example 95 Synthesis of Compound Represented by Formula II-47

Under the protection of nitrogen, 2-(2-bromo-6-fluoro-4-propylphenyl)propane-2-ol (2.75 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(2-fluoro-6-(1H-benzimidazol-5-yl)-4-propylphenyl)propane-2-ol as an almost white powdery solid of 1.38 g with a yield of 44.2%. EI-MS M/Z 313.4[M⁺].

### Example 96 Synthesis of Compound Represented by Formula II-48

Under the protection of nitrogen,
3-bromo-2-(2-hydroxypropan-2-yl)-5-propanebenzonitrile (2.82 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-hydroxy-3-(1H-benzimidazol-5-yl)-5-propylbenzonitrile as an almost white powdery solid of 0.89 g with a yield of 27.9%. EI-MS M/Z 320.4[M⁺].

### Example 97 Synthesis of Compound Represented by Formula II-49

Under the protection of nitrogen, 1-(2-bromo-4-fluorophenyl)ethane-1-ol (2.19 g, 0.1 mol) and (2-methyl-1H-benzimidazol-5-yl)boronic acid (1.76 g, 0.1 mol) were added to 1,4-dioxane (10 ml), and potassium acetate (1.96 g, 0.2 mol), [1,1-bis(diphenylphosphino)ferrocene]palladium(II) dichloride (0.36 g, 5 mmol) and 1,1'-bis(diphenylphosphino)ferrocene (0.28 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(4-fluoro-2-(2-methyl-1H-benzimidazol-5-yl)phenyl)ethane-1-ol as an almost white powdery solid of 1.04 g with a yield of 38.5%. EI-MS M/Z 271.3[M⁺].

### Example 98 Synthesis of Compound Represented by Formula II-50

Under the protection of nitrogen, 1-(2-bromo-4,6-difluorophenyl)ethane-1-ol (2.37 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(2,4-difluoro-6-(2-methyl-1H-benzimidazol-5-yl)phenyl)ethane-1-ol as an almost white powdery solid of 1.69 g with a yield of 58.4%. EI-MS M/Z 289.3[M⁺].

### Example 99 Synthesis of Compound Represented by Formula II-51

Under the protection of nitrogen, 3-bromo-5-fluoro-2-(1-hydroxyethyl)benzonitrile (2.44 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-(1-hydroxyethyl)-3-(2-methyl-1H-benzimidazol-5-yl)benzonitrile as an almost white powdery solid of 1.09 g with a yield of 36.9%. EI-MS M/Z 296.3[M⁺].

### Example 100 Synthesis of Compound Represented by Formula II-52

Under the protection of nitrogen, 3-bromo-4-(1-hydroxyethyl)benzamide (2.42 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(1-hydroxyethyl)-3-(2-methyl-1H-benzimidazol-5-yl)benzamide as an almost white powdery solid of 0.72 g with a yield of 24.4%. EI-MS M/Z 296.3[M⁺].

### Example 101 Synthesis of Compound Represented by Formula II-53

Under the protection of nitrogen, 3-bromo-5-fluoro-4-(1-hydroxyethyl)benzamide (2.62 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(1-hydroxyethyl)-5-(2-methyl-1H-benzimidazol-5-yl)benzamide as an almost white powdery solid of 0.86 g with a yield of 27.4%. EI-MS M/Z 314.3[M⁺].

### Example 102 Synthesis of Compound Represented by Formula II-54

Under the protection of nitrogen, 3-bromo-5-cyano-4-(1-hydroxyethyl)benzamide (2.69 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(1-hydroxyethyl)-5-(2-methyl-1H-benzimidazol-5-yl)benzamide as an almost white powdery solid of 0.70 g with a yield of 21.8%. EI-MS M/Z 321.4[M⁺].

### Example 103 Synthesis of Compound Represented by Formula II-55

Under the protection of nitrogen,
3-bromo-4-(1-hydroxyethyl)-N,N-dimethylbenzamide (2.72 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(1-hydroxyethyl)-N,N-dimethyl-3-(2-methyl-1H-benzimidazol-5-yl)benzamide as an almost white powdery solid of 1.10 g with a yield of 34.0%. EI-MS M/Z 324.4[M⁺].

### Example 104 Synthesis of Compound Represented by Formula II-56

Under the protection of nitrogen,
3-bromo-5-fluoro-4-(1-hydroxyethyl)-N,N-dimethylbenzamide (2.90 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(1-hydroxyethyl)-N,N-dimethyl-5-(2-methyl-1H-benzimidazol-5-yl)benzamide as an almost white powdery solid of 1.25 g with a yield of 38.9%. EI-MS M/Z 342.4[M⁺].

### Example 105 Synthesis of Compound Represented by Formula II-57

Under the protection of nitrogen,
3-bromo-5-cyano-4-(1-hydroxyethyl)-N,N-dimethylbenzamide (2.97 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(1-hydroxyethyl)-N,N-dimethyl-5-(2-methyl-1H-benzimidazol-5-yl)benzamide as an almost white powdery solid of 1.33 g with a yield of 38.1%. EI-MS M/Z 349.4[M⁺].

### Example 106 Synthesis of Compound Represented by Formula II-58

Under the protection of nitrogen, 3-bromo-4-(1-hydroxyethyl)benzoic acid (2.45 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(1-hydroxyethyl)-3-(2-methyl-1H-benzimidazol-5-yl)benzoic acid as an almost white powdery solid of 0.77 g with a yield of 26.0%. EI-MS M/Z 297.3[M⁺].

### Example 107 Synthesis of Compound Represented by Formula II-59

Under the protection of nitrogen, 3-bromo-5-fluoro-4-(1-hydroxyethyl)benzoic acid (2.63 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(1-hydroxyethyl)-5-(2-methyl-1H-benzimidazol-5-yl)benzoic acid as an almost white powdery solid of 0.68 g with a yield of 21.6%. EI-MS M/Z 315.3[M⁺].

### Example 108 Synthesis of Compound Represented by Formula II-60

Under the protection of nitrogen, 3-bromo-5-cyano-4-(1-hydroxyethyl)benzoic acid (2.70 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(1-hydroxyethyl)-5-(2-methyl-1H-benzimidazol-5-yl)benzoic acid as an almost white powdery solid of 0.71 g with a yield of 22.0%. EI-MS M/Z 322.3[M⁺].

### Example 109 Synthesis of Compound Represented by Formula II-61

Under the protection of nitrogen, methyl 3-bromo-4-(1-hydroxyethyl) benzoate (2.59 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-(1-hydroxyethyl)-3-(2-methyl-1H-benzimidazol-5-yl) benzoate as an almost white powdery solid of 1.55 g with a yield of 47.8%. EI-MS M/Z 311.4[M⁺].

### Example 110 Synthesis of Compound Represented by Formula II-62

Under the protection of nitrogen, methyl 3-bromo-5-fluoro-4-(1-hydroxyethyl) benzoate (2.77 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-(1-hydroxyethyl)-5-(2-methyl-1H-benzimidazol-5-yl) benzoate as an almost white powdery solid of 1.74 g with a yield of 53.0%. EI-MS M/Z 329.3[M⁺].

### Example 111 Synthesis of Compound Represented by Formula II-63

Under the protection of nitrogen, methyl 3-bromo-5-cyano-4-(1-hydroxyethyl) benzoate (2.84 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-(1-hydroxyethyl)-5-(2-methyl-1H-benzimidazol-5-yl) benzoate as an almost white powdery solid of 1.60 g with a yield of 47.7%. EI-MS M/Z 336.4[M⁺].

### Example 112 Synthesis of Compound Represented by Formula II-64

Under the protection of nitrogen, 1-(4-allyl-2-bromophenyl)ethane-1-ol (2.40 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(4-allyl-2-(2-methyl-1H-benzimidazol-5-yl)phenyl)ethane-1-ol as an almost white powdery solid of 0.72 g with a yield of 24.6%. EI-MS M/Z 293.4[M⁺].

### Example 113 Synthesis of Compound Represented by Formula II-65

Under the protection of nitrogen, 1-(4-allyl-2-bromo-6-fluorophenyl)ethane-1-ol (2.59 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(4-allyl-2-fluoro-6-(2-methyl-1H-benzimidazol-5-yl)phenyl)ethane-1-ol as an almost white powdery solid of 0.57 g with a yield of 18.4%. EI-MS M/Z 311.4[M⁺].

### Example 114 Synthesis of Compound Represented by Formula II-66

Under the protection of nitrogen, 5-allyl-3-bromo-2-(1-hydroxyethyl)benzonitrile (2.66 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-2-(1-hydroxyethyl)-3-(2-methyl-1H-benzimidazol-5-yl)benzonitrile as an almost white powdery solid of 0.66 g with a yield of 20.7%. EI-MS M/Z 318.4[M⁺].

### Example 115 Synthesis of Compound Represented by Formula II-67

Under the protection of nitrogen, 2-(3-bromo-(1-hydroxyethyl)phenyl)acetonitrile (2.40 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-(1-hydroxyethyl)-3-(2-methyl-1H-benzimidazol-5-yl)phenyl)acetonitrile as an almost white powdery solid of 1.02 g with a yield of 35.9%. EI-MS M/Z 292.4[M⁺].

### Example 116 Synthesis of Compound Represented by Formula II-68

Under the protection of nitrogen,
2-(3-bromo-5-fluoro-4-(1-hydroxyethyl)phenyl)acetonitrile (2.58 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-(1-hydroxyethyl)-5-(2-methyl-1H-benzimidazol-5-yl)phenyl)acetonitrile as an almost white powdery solid of 1.02 g with a yield of 33.0%. EI-MS M/Z 310.3[M⁺].

### Example 117 Synthesis of Compound Represented by Formula II-69

Under the protection of nitrogen,
3-bromo-5-cyanomethyl-2-(1-hydroxyethyl)benzonitrile (2.65 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-(1-hydroxyethyl)-3-(2-methyl-1H-benzimidazol-5-yl)benzonitrile as an almost white powdery solid of 0.84 g with a yield of 26.7%. EI-MS M/Z 317.4[M⁺].

### Example 118 Synthesis of Compound Represented by Formula II-70

Under the protection of nitrogen, 1-(2-bromo-4-propylphenyl)ethane-1-ol (2.43 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(2-(2-methyl-1H-benzimidazol-5-yl)-4-propylphenyl)ethane-1-ol as an almost white powdery solid of 1.58 g with a yield of 53.7%. EI-MS M/Z 295.4[M⁺].

### Example 119 Synthesis of Compound Represented by Formula II-71

Under the protection of nitrogen, 1-(2-bromo-6-fluoro-4-propylphenyl)ethane-2-ol (2.61 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(2-fluoro-6-(2-methyl-1H-benzimidazol-5-yl)-4-propylphenyl)ethane-1-ol as an almost white powdery solid of 1.19 g with a yield of 38.0%. EI-MS M/Z 313.4[M⁺].

### Example 120 Synthesis of Compound Represented by Formula II-72

Under the protection of nitrogen, 3-bromo-2-(1-hydroxyethyl)-5-propanebenzonitrile (2.68 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(1-hydroxyethyl)-3-(2-methyl-1H-benzimidazol-5-yl)-5-propylbenzonitrile as an almost white powdery solid of 1.19 g with a yield of 37.3%. EI-MS M/Z 320.4[M⁺].

### Example 121 Synthesis of Compound Represented by Formula II-73

Under the protection of nitrogen, 1-(2-bromo-4-fluorophenyl)ethane-1-ol (2.19 g, 0.1 mol) and (1H-benzimidazol-5-yl)boronic acid (1.62 g, 0.1 mol) were added to 1,4-dioxane (10 ml), and potassium acetate (1.96 g, 0.2 mol), [1,1-bis(diphenylphosphino)ferrocene]palladium(II) dichloride (0.36 g, 5 mmol) and 1,1'-bis(diphenylphosphino)ferrocene (0.28 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(4-fluoro-2-(1H-benzimidazol-5-yl)phenyl)ethane-1-ol as an almost white powdery solid of 1.33 g with a yield of 51.9%. EI-MS M/Z 257.3[M⁺].

### Example 122 Synthesis of Compound Represented by Formula II-74

Under the protection of nitrogen, 1-(2-bromo-4,6-difluorophenyl)ethane-1-ol (2.37 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(2,4-difluoro-6-(1H-benzimidazol-5-yl)phenyl)ethane-1-ol as an almost white powdery solid of 1.58 g with a yield of 57.6%. EI-MS M/Z 275.3[M⁺].

### Example 123 Synthesis of Compound Represented by Formula II-75

Under the protection of nitrogen, 3-bromo-5-fluoro-2-(1-hydroxyethyl)benzonitrile (2.44 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-(1-hydroxyethyl)-3-(1H-benzimidazol-5-yl)benzonitrile as an almost white powdery solid of 0.89 g with a yield of 31.6%. EI-MS M/Z 282.3[M⁺].

### Example 124 Synthesis of Compound Represented by Formula II-76

Under the protection of nitrogen, 3-bromo-4-(1-hydroxyethyl)benzamide (2.42 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(1-hydroxyethyl)-3-(1H-benzimidazol-5-yl)benzamide as an almost white powdery solid of 0.71 g with a yield of 25.2%. EI-MS M/Z 282.3[M⁺].

### Example 125 Synthesis of Compound Represented by Formula II-77

Under the protection of nitrogen, 3-bromo-5-fluoro-4-(1-hydroxyethyl)benzamide (2.62 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(1-hydroxyethyl)-5-(1H-benzimidazol-5-yl)benzamide as an almost white powdery solid of 0.81 g with a yield of 27.1%. EI-MS M/Z 300.3[M⁺].

### Example 126 Synthesis of Compound Represented by Formula II-78

Under the protection of nitrogen, 3-bromo-5-cyano-4-(1-hydroxyethyl)benzamide (2.69 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(1-hydroxyethyl)-5-(1H-benzimidazol-5-yl)benzamide as an almost white powdery solid of 0.61 g with a yield of 19.9%. EI-MS M/Z 307.3[M⁺].

### Example 127 Synthesis of Compound Represented by Formula II-79

Under the protection of nitrogen,
3-bromo-4-(1-hydroxyethyl)-N,N-dimethylbenzamide (2.72 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(1-hydroxyethyl)-N,N-dimethyl-3-(1H-benzimidazol-5-yl)benzamide as an almost white powdery solid of 1.21 g with a yield of 39.1%. EI-MS M/Z 310.4[M⁺].

### Example 128 Synthesis of Compound Represented by Formula II-80

Under the protection of nitrogen,
3-bromo-5-fluoro-4-(1-hydroxyethyl)-N,N-dimethylbenzamide (2.90 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(1-hydroxyethyl)-N,N-dimethyl-5-(1H-benzimidazol-5-yl)benzamide as an almost white powdery solid of 1.22 g with a yield of 37.3%. EI-MS M/Z 328.4[M⁺].

### Example 129 Synthesis of Compound Represented by Formula II-81

Under the protection of nitrogen,
3-bromo-5-cyano-4-(1-hydroxyethyl)-N,N-dimethylbenzamide (2.97 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(1-hydroxyethyl)-N,N-dimethyl-5-(1H-benzimidazol-5-yl)benzamide as an almost white powdery solid of 1.22 g with a yield of 36.5%. EI-MS M/Z 335.4[M⁺].

### Example 130 Synthesis of Compound Represented by Formula II-82

Under the protection of nitrogen, 3-bromo-4-(1-hydroxyethyl)benzoic acid (2.45 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(1-hydroxyethyl)-3-(1H-benzimidazol-5-yl)benzoic acid as an almost white powdery solid of 0.71 g with a yield of 25.2%. EI-MS M/Z 283.3[M⁺].

### Example 131 Synthesis of Compound Represented by Formula II-83

Under the protection of nitrogen, 3-bromo-5-fluoro-4-(1-hydroxyethyl)benzoic acid (2.63 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(1-hydroxyethyl)-5-(1H-benzimidazol-5-yl)benzoic acid as an almost white powdery solid of 0.60 g with a yield of 20.0%. EI-MS M/Z 301.3[M⁺].

### Example 132 Synthesis of Compound Represented by Formula II-84

Under the protection of nitrogen, 3-bromo-5-cyano-(1-hydroxyethyl)benzoic acid (2.70 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(1-hydroxyethyl)-5-(1H-benzimidazol-5-yl)benzoic acid as an almost white powdery solid of 0.62 g with a yield of 20.2%. EI-MS M/Z 308.3[M⁺].

### Example 133 Synthesis of Compound Represented by Formula II-85

Under the protection of nitrogen, methyl 3-bromo-4-(1-hydroxyethyl) benzoate (2.59 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-(1-hydroxyethyl)-3-(1H-benzimidazol-5-yl) benzoate as an almost white powdery solid of 1.59 g with a yield of 53.6%. EI-MS M/Z 297.3[M⁺].

### Example 134 Synthesis of Compound Represented by Formula II-86

Under the protection of nitrogen, methyl 3-bromo-5-fluoro-4-(1-hydroxyethyl) benzoate (2.77 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-(1-hydroxyethyl)-5-(1H-benzimidazol-5-yl) benzoate as an almost white powdery solid of 1.54 g with a yield of 49.0%. EI-MS M/Z 315.3[M⁺].

### Example 135 Synthesis of Compound Represented by Formula II-87

Under the protection of nitrogen, methyl 3-bromo-5-cyano-4-(1-hydroxyethyl) benzoate (2.84 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-(1-hydroxyethyl)-5-(1H-benzimidazol-5-yl) benzoate as an almost white powdery solid of 1.68 g with a yield of 52.3%. EI-MS M/Z 322.3[M⁺].

### Example 136 Synthesis of Compound Represented by Formula II-88

Under the protection of nitrogen, 1-(4-allyl-2-bromophenyl)ethane-1-ol (2.40 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(4-allyl-2-(1H-benzimidazol-5-yl)phenyl)ethane-1-ol as an almost white powdery solid of 0.77 g with a yield of 27.7%. EI-MS M/Z 279.4[M⁺].

### Example 137 Synthesis of Compound Represented by Formula II-89

Under the protection of nitrogen, 1-(4-allyl-2-bromo-6-fluorophenyl)ethane-1-ol (2.59 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(4-allyl-2-fluoro-6-(1H-benzimidazol-5-yl)phenyl)ethane-1-ol as an almost white powdery solid of 0.51 g with a yield of 17.2%. EI-MS M/Z 297.4[M⁺].

### Example 138 Synthesis of Compound Represented by Formula II-90

Under the protection of nitrogen, 5-allyl-3-bromo-2-(1-hydroxyethyl)benzonitrile (2.66 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-2-(1-hydroxyethyl)-3-(1H-benzimidazol-5-yl)benzonitrile as an almost white powdery solid of 0.69 g with a yield of 22.7%. EI-MS M/Z 304.4[M⁺].

### Example 139 Synthesis of Compound Represented by Formula II-91

Under the protection of nitrogen, 2-(3-bromo-(1-hydroxyethyl)phenyl)acetonitrile (2.40 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-(1-hydroxyethyl)-3-(1H-benzimidazol-5-yl)phenyl)acetonitrile as an almost white powdery solid of 0.85 g with a yield of 30.6%. EI-MS M/Z 278.3[M⁺].

### Example 140 Synthesis of Compound Represented by Formula II-92

Under the protection of nitrogen,
2-(3-bromo-5-fluoro-4-(1-hydroxyethyl)phenyl)acetonitrile (2.58 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-(1-hydroxyethyl)-5-(1H-benzimidazol-5-yl)phenyl)acetonitrile as an almost white powdery solid of 1.24 g with a yield of 42.0%. EI-MS M/Z 296.3[M⁺].

### Example 141 Synthesis of Compound Represented by Formula II-93

Under the protection of nitrogen,
3-bromo-5-cyanomethyl-2-(1-hydroxyethyl)benzonitrile (2.65 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-(1-hydroxyethyl)-3-(1H-benzimidazol-5-yl)benzonitrile as an almost white powdery solid of 0.89 g with a yield of 29.4%. EI-MS M/Z 303.3[M⁺].

### Example 142 Synthesis of Compound Represented by Formula II-94

Under the protection of nitrogen, 1-(2-bromo-4-propylphenyl)ethane-1-ol (2.43 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(2-(1H-benzimidazol-5-yl)-4-propylphenyl)ethane-1-ol as an almost white powdery solid of 1.55 g with a yield of 55.3%. EI-MS M/Z 281.4[M⁺].

### Example 143 Synthesis of Compound Represented by Formula II-95

Under the protection of nitrogen, 1-(2-bromo-6-fluoro-4-propylphenyl)ethane-2-ol (2.61 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 1-(2-fluoro-6-(1H-benzimidazol-5-yl)-4-propylphenyl)ethane-1-ol as an almost white powdery solid of 1.06 g with a yield of 35.5%. EI-MS M/Z 299.4[M⁺].

### Example 144 Synthesis of Compound Represented by Formula II-96

Under the protection of nitrogen, 3-bromo-2-(1-hydroxyethyl)-5-propanebenzonitrile (2.68 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(1-hydroxyethyl)-3-(1H-benzimidazol-5-yl)-5-propylbenzonitrile as an almost white powdery solid of 1.02 g with a yield of 33.4%. EI-MS M/Z 306.4[M⁺].

### Example 145 Synthesis of Compound Represented by Formula II-97

Under the protection of nitrogen, (2-bromo-4-fluorophenyl)methanol (2.05 g, 0.1 mol) and (2-methyl-1H-benzimidazol-5-yl)boronic acid (1.76 g, 0.1 mol) were added to 1,4-dioxane (10 ml), and potassium acetate (1.96 g, 0.2 mol), [1,1-bis(diphenylphosphino)ferrocene]palladium(II) dichloride (0.36 g, 5 mmol) and 1,1'-bis(diphenylphosphino)ferrocene (0.28 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (4-fluoro-2-(2-methyl-1H-benzimidazol-5-yl)phenyl)methanol as an almost white powdery solid of 1.11 g with a yield of 43.3%. EI-MS M/Z 257.3[M⁺].

### Example 146 Synthesis of Compound Represented by Formula II-98

Under the protection of nitrogen, (2-bromo-4,6-difluorophenyl)methanol (2.23 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (2,4-difluoro-6-(2-methyl-1H-benzimidazol-5-yl)phenyl)methanol as an almost white powdery solid of 1.39 g with a yield of 50.7%. EI-MS M/Z 275.3[M⁺].

### Example 147 Synthesis of Compound Represented by Formula II-99

Under the protection of nitrogen, 3-bromo-5-fluoro-2-(hydroxymethyl)benzonitrile (2.30 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-(hydroxymethyl)-3-(2-methyl-1H-benzimidazol-5-yl)benzonitrile as an almost white powdery solid of 1.01 g with a yield of 35.9%. EI-MS M/Z 282.3[M⁺].

### Example 148 Synthesis of Compound Represented by Formula II-100

Under the protection of nitrogen, 3-bromo-4-(hydroxymethyl)benzamide (2.30 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(hydroxymethyl)-3-(2-methyl-1H-benzimidazol-5-yl)benzamide as an almost white powdery solid of 0.66 g with a yield of 23.5%. EI-MS M/Z 282.3[M⁺].

### Example 149 Synthesis of Compound Represented by Formula II-101

Under the protection of nitrogen, 3-bromo-5-fluoro-4-(hydroxymethyl)benzamide (2.48 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(hydroxymethyl)-5-(2-methyl-1H-benzimidazol-5-yl)benzamide as an almost white powdery solid of 0.56 g with a yield of 18.7%. EI-MS M/Z 300.3[M⁺].

### Example 150 Synthesis of Compound Represented by Formula 11-102

Under the protection of nitrogen, 3-bromo-5-cyano-4-(hydroxymethyl)benzamide (2.55 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(hydroxymethyl)-5-(2-methyl-1H-benzimidazol-5-yl)benzamide as an almost white powdery solid of 0.88 g with a yield of 28.7%. EI-MS M/Z 307.3[M⁺].

### Example 151 Synthesis of Compound Represented by Formula 11-103

Under the protection of nitrogen,
3-bromo-4-(hydroxymethyl)-N,N-dimethylbenzamide (2.58 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(hydroxymethyl)-N,N-dimethyl-3-(2-methyl-1H-benzimidazol-5-yl)benzamide as an almost white powdery solid of 1.82 g with a yield of 58.8%. EI-MS M/Z 310.4[M⁺].

### Example 152 Synthesis of Compound Represented by Formula 11-104

Under the protection of nitrogen,
3-bromo-5-fluoro-4-(hydroxymethyl)-N,N-dimethylbenzamide (2.76 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(hydroxymethyl)-N,N-dimethyl-5-(2-methyl-1H-benzimidazol-5-yl)benzamide as an almost white powdery solid of 1.45 g with a yield of 44.3%. EI-MS M/Z 328.4[M⁺].

### Example 153 Synthesis of Compound Represented by Formula II-105

Under the protection of nitrogen,
3-bromo-5-cyano-4-(hydroxymethyl)-N,N-dimethylbenzamide (2.83 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(hydroxymethyl)-N,N-dimethyl-5-(2-methyl-1H-benzimidazol-5-yl)benzamide as an almost white powdery solid of 1.16 g with a yield of 34.7%. EI-MS M/Z 335.4[M⁺].

### Example 154 Synthesis of Compound Represented by Formula II-106

Under the protection of nitrogen, 3-bromo-4-(hydroxymethyl)benzoic acid (2.31 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(hydroxymethyl)-3-(2-methyl-1H-benzimidazol-5-yl)benzoic acid as an almost white powdery solid of 0.57 g with a yield of 20.2%. EI-MS M/Z 283.3[M⁺].

### Example 155 Synthesis of Compound Represented by Formula II-107

Under the protection of nitrogen, 3-bromo-5-fluoro-4-(hydroxymethyl)benzoic acid (2.49 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(hydroxymethyl)-5-(2-methyl-1H-benzimidazol-5-yl)benzoic acid as an almost white powdery solid of 0.53 g with a yield of 17.6%. EI-MS M/Z 301.3[M⁺].

### Example 156 Synthesis of Compound Represented by Formula II-108

Under the protection of nitrogen, 3-bromo-5-cyano-4-(hydroxymethyl)benzoic acid (2.56 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(hydroxymethyl)-5-(2-methyl-1H-benzimidazol-5-yl)benzoic acid as an almost white powdery solid of 0.63 g with a yield of 20.5%. EI-MS M/Z 308.3[M⁺].

### Example 157 Synthesis of Compound Represented by Formula II-109

Under the protection of nitrogen, methyl 3-bromo-4-(hydroxymethyl) benzoate (2.45 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-(hydroxymethyl)-3-(2-methyl-1H-benzimidazol-5-yl) benzoate as an almost white powdery solid of 1.85 g with a yield of 62.4%. EI-MS M/Z 297.3[M⁺].

### Example 158 Synthesis of Compound Represented by Formula II-110

Under the protection of nitrogen, methyl 3-bromo-5-fluoro-4-(hydroxymethyl) benzoate (2.63 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-(hydroxymethyl)-5-(2-methyl-1H-benzimidazol-5-yl) benzoate as an almost white powdery solid of 1.77 g with a yield of 56.3%. EI-MS M/Z 315.3[M⁺].

### Example 159 Synthesis of Compound Represented by Formula II-111

Under the protection of nitrogen, methyl 3-bromo-5-cyano-4-(hydroxymethyl) benzoate (2.70 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-(hydroxymethyl)-5-(2-methyl-1H-benzimidazol-5-yl) benzoate as an almost white powdery solid of 1.69 g with a yield of 52.6%. EI-MS M/Z 322.4[M⁺].

### Example 160 Synthesis of Compound Represented by Formula 11-112

Under the protection of nitrogen, (4-allyl-2-bromophenyl)methanol (2.27 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (4-allyl-2-(2-methyl-1H-benzimidazol-5-yl)phenyl)methanol as an almost white powdery solid of 0.42 g with a yield of 14.1%. EI-MS M/Z 279.4[M⁺].

### Example 161 Synthesis of Compound Represented by Formula 11-113

Under the protection of nitrogen, (4-allyl-2-bromo-6-fluorophenyl)methanol (2.45 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (4-allyl-2-fluoro-6-(2-methyl-1H-benzimidazol-5-yl)phenyl)methanol as an almost white powdery solid of 0.41 g with a yield of 13.8%. EI-MS M/Z 297.4[M⁺].

### Example 162 Synthesis of Compound Represented by Formula 11-114

Under the protection of nitrogen, 5-allyl-3-bromo-2-(hydroxymethyl)benzonitrile (2.52 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-2-(hydroxymethyl)-3-(2-methyl-1H-benzimidazol-5-yl)benzonitrile as an almost white powdery solid of 0.51 g with a yield of 16.8%. EI-MS M/Z 304.4[M⁺].

### Example 163 Synthesis of Compound Represented by Formula 11-115

Under the protection of nitrogen, 2-(3-bromo-4-(hydroxymethyl)phenyl)acetonitrile (2.26 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-(hydroxymethyl)-3-(2-methyl-1H-benzimidazol-5-yl)phenyl)acetonitrile as an almost white powdery solid of 0.88 g with a yield of 31.7%. EI-MS M/Z 278.4[M⁺].

### Example 164 Synthesis of Compound Represented by Formula 11-116

Under the protection of nitrogen,
2-(3-bromo-5-fluoro-4-(hydroxymethyl)phenyl)acetonitrile (2.44 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-(hydroxymethyl)-5-(2-methyl-1H-benzimidazol-5-yl)phenyl)acetonitrile as an almost white powdery solid of 1.22 g with a yield of 41.3%. EI-MS M/Z 296.3[M⁺].

### Example 165 Synthesis of Compound Represented by Formula II-117

Under the protection of nitrogen,
3-bromo-5-cyanomethyl-2-(hydroxymethyl)benzonitrile (2.51 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-(hydroxymethyl)-3-(2-methyl-1H-benzimidazol-5-yl)benzonitrile as an almost white powdery solid of 0.82 g with a yield of 27.1%. EI-MS M/Z 303.3[M⁺].

### Example 166 Synthesis of Compound Represented by Formula II-118

Under the protection of nitrogen, (2-bromo-4-propylphenyl)methanol (2.29 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (2-(2-methyl-1H-benzimidazol-5-yl)-4-propylphenyl)methanol as an almost white powdery solid of 1.58 g with a yield of 53.7%. EI-MS M/Z 281.4[M⁺].

### Example 167 Synthesis of Compound Represented by Formula 11-119

Under the protection of nitrogen, (2-bromo-6-fluoro-4-propylphenyl)methanol (2.47 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (2-fluoro-6-(2-methyl-1H-benzimidazol-5-yl)-4-propylphenyl)methanol as an almost white powdery solid of 1.06 g with a yield of 35.5%. EI-MS M/Z 299.4[M⁺].

### Example 168 Synthesis of Compound Represented by Formula 11-120

Under the protection of nitrogen, 3-bromo-2-(hydroxymethyl)-5-propanebenzonitrile (2.54 g, 0.01 mol) and (2-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(hydroxymethyl)-3-(2-methyl-1H-benzimidazol-5-yl)-5-propylbenzonitrile as an almost white powdery solid of 1.12 g with a yield of 36.7%. EI-MS M/Z 306.4[M⁺].

### Example 169 Synthesis of Compound Represented by Formula II-121

Under the protection of nitrogen, (2-bromo-4-fluorophenyl)methanol (2.05 g, 0.1 mol) and (1H-benzimidazol-5-yl)boronic acid (1.62 g, 0.1 mol) were added to 1,4-dioxane (10 ml), and potassium acetate (1.96 g, 0.2 mol), [1,1-bis(diphenylphosphino)ferrocene]palladium(II) dichloride (0.36 g, 5 mmol) and 1,1'-bis(diphenylphosphino)ferrocene (0.28 g, 5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (4-fluoro-2-(1H-benzimidazol-5-yl)phenyl)methanol as an almost white powdery solid of 1.24 g with a yield of 51.2%. EI-MS M/Z 243.3[M⁺].

### Example 170 Synthesis of Compound Represented by Formula 11-122

Under the protection of nitrogen, (2-bromo-4,6-difluorophenyl)methanol (2.23 g, 0.01 mol) and (1H-benzimidazol-5-yl)boronic acid (1.62 g, 0.1 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (2,4-difluoro-6-(1H-benzimidazol-5-yl)phenyl)methanol as an almost white powdery solid of 1.52 g with a yield of 58.4%. EI-MS M/Z 261.2[M⁺].

### Example 171 Synthesis of Compound Represented by Formula 11-123

Under the protection of nitrogen, 3-bromo-5-fluoro-2-(hydroxymethyl)benzonitrile (2.30 g, 0.01 mol) and (1H-benzimidazol-5-yl)boronic acid (1.62 g, 0.1 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-fluoro-2-(hydroxymethyl)-3-(1H-benzimidazol-5-yl)benzonitrile as an almost white powdery solid of 1.13 g with a yield of 42.3%. EI-MS M/Z 268.3[M⁺].

### Example 172 Synthesis of Compound Represented by Formula 11-124

Under the protection of nitrogen, 3-bromo-4-(hydroxymethyl)benzamide (2.30 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(hydroxymethyl)-3-(1H-benzimidazol-5-yl)benzamide as an almost white powdery solid of 0.58 g with a yield of 21.7%. EI-MS M/Z 268.3[M⁺].

### Example 173 Synthesis of Compound Represented by Formula 11-125

Under the protection of nitrogen, 3-bromo-5-fluoro-4-(hydroxymethyl)benzamide (2.48 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(hydroxymethyl)-5-(1H-benzimidazol-5-yl)benzamide as an almost white powdery solid of 0.65 g with a yield of 22.8%. EI-MS M/Z 286.3[M⁺].

### Example 174 Synthesis of Compound Represented by Formula 11-126

Under the protection of nitrogen, 3-bromo-5-cyano-4-(hydroxymethyl)benzamide (2.55 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(hydroxymethyl)-5-(1H-benzimidazol-5-yl)benzamide as an almost white powdery solid of 0.83 g with a yield of 28.4%. EI-MS M/Z 293.3[M⁺].

### Example 175 Synthesis of Compound Represented by Formula 11-127

Under the protection of nitrogen,
3-bromo-4-(hydroxymethyl)-N,N-dimethylbenzamide (2.58 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(hydroxymethyl)-N,N-dimethyl-3-(1H-benzimidazol-5-yl)benzamide as an almost white powdery solid of 1.12 g with a yield of 37.9%. EI-MS M/Z 296.3[M⁺].

### Example 176 Synthesis of Compound Represented by Formula 11-128

Under the protection of nitrogen,
3-bromo-5-fluoro-4-(hydroxymethyl)-N,N-dimethylbenzamide (2.76 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(hydroxymethyl)-N,N-dimethyl-5-(1H-benzimidazol-5-yl)benzamide as an almost white powdery solid of 1.05 g with a yield of 33.5%. EI-MS M/Z 314.3[M⁺].

### Example 177 Synthesis of Compound Represented by Formula 11-129

Under the protection of nitrogen,
3-bromo-5-cyano-4-(hydroxymethyl)-N,N-dimethylbenzamide (2.83 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(hydroxymethyl)-N,N-dimethyl-5-(1H-benzimidazol-5-yl)benzamide as an almost white powdery solid of 1.03 g with a yield of 32.1%. EI-MS M/Z 321.4[M⁺].

### Example 178 Synthesis of Compound Represented by Formula II-130

Under the protection of nitrogen, 3-bromo-4-(hydroxymethyl)benzoic acid (2.31 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-(hydroxymethyl)-3-(1H-benzimidazol-5-yl)benzoic acid as an almost white powdery solid of 0.37 g with a yield of 13.8%. EI-MS M/Z 269.3[M⁺].

### Example 179 Synthesis of Compound Represented by Formula 11-131

Under the protection of nitrogen, 3-bromo-5-fluoro-4-(hydroxymethyl)benzoic acid (2.49 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-fluoro-4-(hydroxymethyl)-5-(1H-benzimidazol-5-yl)benzoic acid as an almost white powdery solid of 0.63 g with a yield of 22.0%. EI-MS M/Z 287.3[M⁺].

### Example 180 Synthesis of Compound Represented by Formula 11-132

Under the protection of nitrogen, 3-bromo-5-cyano-(hydroxymethyl)benzoic acid (2.56 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 3-cyano-4-(hydroxymethyl)-5-(1H-benzimidazol-5-yl)benzoic acid as an almost white powdery solid of 0.33 g with a yield of 11.3%. EI-MS M/Z 294.3[M⁺].

### Example 181 Synthesis of Compound Represented by Formula 11-133

Under the protection of nitrogen, methyl 3-bromo-4-(hydroxymethyl) benzoate (2.45 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 4-(hydroxymethyl)-3-(1H-benzimidazol-5-yl) benzoate as an almost white powdery solid of 1.63 g with a yield of 57.7%. EI-MS M/Z 283.3[M⁺].

### Example 182 Synthesis of Compound Represented by Formula 11-134

Under the protection of nitrogen, methyl 3-bromo-5-fluoro-4-(hydroxymethyl) benzoate (2.63 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-fluoro-4-(hydroxymethyl)-5-(1H-benzimidazol-5-yl) benzoate as an almost white powdery solid of 1.68 g with a yield of 55.9%. EI-MS M/Z 301.3[M⁺].

### Example 183 Synthesis of Compound Represented by Formula 11-135

Under the protection of nitrogen, methyl 3-bromo-5-cyano-4-(hydroxymethyl) benzoate (2.70 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain methyl 3-cyano-4-(hydroxymethyl)-5-(1H-benzimidazol-5-yl) benzoate as an almost white powdery solid of 1.32 g with a yield of 43.0%. EI-MS M/Z 308.3[M⁺].

### Example 184 Synthesis of Compound Represented by Formula 11-136

Under the protection of nitrogen, (4-allyl-2-bromophenyl)methanol (2.27 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (4-allyl-2-(1H-benzimidazol-5-yl)phenyl)methanol as an almost white powdery solid of 0.22 g with a yield of 8.3%. EI-MS M/Z 265.3[M⁺].

### Example 185 Synthesis of Compound Represented by Formula 11-137

Under the protection of nitrogen, (4-allyl-2-bromo-6-fluorophenyl)methanol (2.45 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (4-allyl-2-fluoro-6-(1H-benzimidazol-5-yl)phenyl)methanol as an almost white powdery solid of 0.36 g with a yield of 12.8%. EI-MS M/Z 283.3[M⁺].

### Example 186 Synthesis of Compound Represented by Formula 11-138

Under the protection of nitrogen, 5-allyl-3-bromo-2-(hydroxymethyl)benzonitrile (2.52 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-allyl-2-(hydroxymethyl)-3-(1H-benzimidazol-5-yl)benzonitrile as an almost white powdery solid of 0.38 g with a yield of 13.1%. EI-MS M/Z 290.3[M⁺].

### Example 187 Synthesis of Compound Represented by Formula 11-139

Under the protection of nitrogen, 2-(3-bromo-4-(hydroxymethyl)phenyl)acetonitrile (2.26 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(4-(hydroxymethyl)-3-(1H-benzimidazol-5-yl)phenyl)acetonitrile as an almost white powdery solid of 1.15 g with a yield of 43.7%. EI-MS M/Z 264.3[M⁺].

### Example 188 Synthesis of Compound Represented by Formula 11-140

Under the protection of nitrogen,
2-(3-bromo-5-fluoro-4-(hydroxymethyl)phenyl)acetonitrile (2.44 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110°C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(3-fluoro-4-(hydroxymethyl)-5-(1H-benzimidazol-5-yl)phenyl)acetonitrile as an almost white powdery solid of 1.06 g with a yield of 37.7%. EI-MS M/Z 282.3[M⁺].

### Example 189 Synthesis of Compound Represented by Formula II-141

Under the protection of nitrogen,
3-bromo-5-cyanomethyl-2-(hydroxymethyl)benzonitrile (2.51 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-cyanomethyl-2-(hydroxymethyl)-3-(1H-benzimidazol-5-yl)benzonitrile as an almost white powdery solid of 1.15 g with a yield of 39.9%. EI-MS M/Z 289.3[M⁺].

### Example 190 Synthesis of Compound Represented by Formula 11-142

Under the protection of nitrogen, (2-bromo-4-propylphenyl)methanol (2.29 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (2-(1H-benzimidazol-5-yl)-4-propylphenyl)methanol as an almost white powdery solid of 1.06 g with a yield of 39.8%. EI-MS M/Z 267.3[M⁺].

### Example 191 Synthesis of Compound Represented by Formula II-143

Under the protection of nitrogen, (2-bromo-6-fluoro-4-propylphenyl)methanol (2.47 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain (2-fluoro-6-(1H-benzimidazol-5-yl)-4-propylphenyl)methanol as an almost white powdery solid of 1.23 g with a yield of 42.3%. EI-MS M/Z 285.3[M⁺].

### Example 192 Synthesis of Compound Represented by Formula II-144

Under the protection of nitrogen, 3-bromo-2-(hydroxymethyl)-5-propanebenzonitrile (2.54 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(hydroxymethyl)-3-(1H-benzimidazol-5-yl)-5-propylbenzonitrile as an almost white powdery solid of 1.52 g with a yield of 52.2%. EI-MS M/Z 292.4[M⁺].

### Example 193 Synthesis of Formula I-24-A

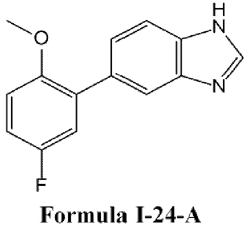

Under the protection of nitrogen, 2-bromo-4-fluoro-1-anisole (2.05 g, 0.01 mol) and (1H-benzimidazol-5-yl)boronic acid (1.62 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 5-(5-fluoro-2-methoxyphenyl)-1H-benzimidazole as an almost white powdery solid of 1.55 g with a yield of 64.0%. EI-MS M/Z 243.3[M⁺].

### Example 194 Synthesis of Formula I-24-B

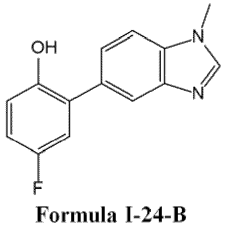

Under the protection of nitrogen, 2-bromo-4-fluorophenol (1.91 g, 0.01 mol) and (1-methyl-1H-benzimidazol-5-yl)boronic acid (1.76 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 4-fluoro-2-(1-methyl-1H-benzimidazol-5-yl)phenol as an almost white powdery solid of 1.15 g with a yield of 47.5%. EI-MS M/Z 243.3[M⁺].

### Example 195 Synthesis of Formula I-24-C

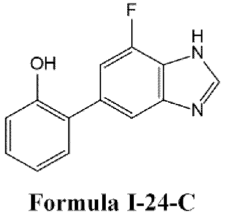

Under the protection of nitrogen, 2-bromophenol (1.73 g, 0.01 mol) and (7-fluoro-1H-benzimidazol-5-yl)boronic acid (1.80 g, 0.01 mol) were added to 1,4-dioxane (10 ml), and potassium acetate (1.96 g, 0.02 mol), [1,1-bis(diphenylphosphino)ferrocene]palladium(II) dichloride (0.36 g, 0.5 mmol) and 1,1'-bis(diphenylphosphino)ferrocene (0.28 g, 0.5 mmol) were added. The mixture was heated to 110 °C, and a reaction was conducted for 2 h. After the reaction was finished, the reaction solution was cooled, water/ethyl acetate was added for extraction, an organic phase was concentrated to dryness and subjected to column chromatography, and ethyl acetate/petroleum ether was used as an eluent to obtain 2-(7-fluoro-1H-benzimidazol-5-yl)phenol as an almost white powdery solid of 1.02 g with a yield of 44.69%. EI-MS M/Z 229.2[M⁺].

### Example 196 Biological Activity Test after Injury of Primary Cardiomyocytes of Suckling Rats

### Test Method

Primary cardiomyocytes of suckling rats were isolated and cultured and inoculated on a cell culture plate. A drug stock solution was taken and separately added to cell wells according to a certain dilution ratio, and a final concentration was 10 µM. After half hour of drug action, a phenylephrine (PE) stock solution was added, and a final concentration of PE was 20 µM. After 48 hours of PE action, cell samples were collected for real-time quantitative PCR assay or cell area measurement.

**Separation and plating of cardiomyocytes of rats:** a certain number of SPF suckling rats were taken and placed in a clean bench according to an actual requirement for the number of cells, thoracic cavities were opened by scissors, hearts were separated, and the hearts were cut into tissue fragments with sterilized surgical scissors; Collagenase Type II (manufacturer: Sigma, article No.: C2-28-100MG) was added to a D-HANKS balanced salt buffer to prepare a 5 µg/mL enzyme digestion solution, and the tissue fragments of hearts were continuously digested for three times in a 37 °C constant-temperature water bath box; about 2 mL of the enzyme digestion solution was added for the first time, a supernatant was discarded after digestion, centrifugation and precipitation of cardiomyocytes and tissue fragments, about 4 mL of the enzyme digestion solution was independently added for the second time and the third time to continue digestion, a supernatant was remained each time, DMEM containing 10% FBS with the same volume as the digestion solution in the supernatant was immediately added to neutralize digestive enzymes, the supernatants for the second time and the third time were combined and centrifuged, a supernatant was discarded, an appropriate amount of DMEM containing 10% FBS was added to resuspend the cardiomyocytes, the cardiomyocytes were inoculated in a 100 mm culture plate and subjected to differential adhesion in a constant-temperature cell incubator for 2 h, and a supernatant was taken for cell count.

**Area measurement of cardiomyocytes of rats:** a 24-well plate precoated with 1% gelatin was taken and plated at 1.0×10³ cells/well, 500 µL of a 10% FBS+DMEM medium was contained in each well, and cells were cultured in a 37 °C constant-temperature cell incubator for 24 h; different compounds were independently prepared into a 100 mM mother liquor with DMSO and gradient diluted with a 10% FBS+DMEM medium to prepare a 10 µM final solution of the compound containing 0.5% DMSO; the medium in the 24-well plate was discarded and replaced with a 500 µL/well medium containing a 10 µM compound, and three replicate wells and a negative control well were set for each compound; after 0.5 hours of drug action, PE (manufacturer: Meilun Biotechnology; article No.: MB1602) with a final concentration of 20 µM was added to each well, and no PE was added to the negative control wells; after 48 hours of PE action, at least five fields of view were randomly selected from each well under an inverted microscope (manufacturer: Nikon; model: Eclipse TS100), and the cell area data was calculated by Image J software; the area of the cells in the negative control wells was set to 1, the cell area hypertrophy multiples of the PE wells and each administration well were calculated separately, and statistical analysis was performed using the One-Way ANOVA method.

### Real-Time Quantitative PCR

A 24-well plate precoated with 1% gelatin was taken and plated at 1×10⁵ cells/well, 500 µL of a 10% FBS+DMEM medium was contained in each well, and cells were cultured in a 37 °C constant-temperature cell incubator for 24 h. Different compounds were independently prepared into a 100 mM mother liquor with DMSO and gradient diluted with a 10% FBS+DMEM medium to prepare a 10 µM final solution of the compound containing 0.5% DMSO. The medium in the 24-well plate was discarded and replaced with a 500 µL/well medium containing a 10 µM compound, and three replicate wells and a negative control well were set for each compound. After 0.5 hours of drug action, PE (manufacturer: Meilun Biotechnology; article No.: MB1602) with a final concentration of 20 µM was added to each well, and no PE was added to the negative control wells; after 48 hours of PE action, sample detection was performed.

Expression levels of genes related to cardiomyocyte hypertrophy was analyzed via real-time quantitative PCR detection of mRNA level of atrial natriuretic peptide (ANP) and myosin heavy chain β (β-MHC). The steps include extraction of total RNA of cells, reverse transcription synthesis of cDNA and real-time quantitative PCR assay.

The real-time quantitative PCR step (note: all relevant primers may be purchased commercially) was completed according to the reaction system and reaction procedure below.
ANP primers:
   forward primer: AGAGACGGCAGTGCTCTAGG (SEQ ID NO: 1)
   reverse primer: CAATCCTACCCCCGAAGCAG (SEQ ID NO: 2)
β-MHC primers:
   forward primer: CTCCAGGGGTGATGGACAAC (SEQ ID NO: 3)
   reverse primer: CGATACCTCTGCCGGAAGTC (SEQ ID NO: 4)
Reaction system: 20 µL

### Reaction procedure:

| Operation Procedure | Temperature | Time |
|---|---|---|
| predenaturation | 95 °C | 30 seconds |

| number of amplification cycles: 40 | | |
|---|---|---|
| denaturation | 95 °C | 3 seconds |
| annealing, extension and fluorescence detection | 60 °C | 30 seconds |

### Summary and analysis of results

(1) Pre-test of cardiomyocyte hypertrophy modeling: before the test, it was necessary to confirm that PE can successfully induce cardiomyocyte hypertrophy.
(2) Drug screening results

Based on real-time quantitative PCR detection of an mRNA level of ANP of cardiomyocytes, a drug with a potential of anti-cardiomyocyte hypertrophy was preliminarily screened and verified with an mRNA level of another hypertrophy gene β-MHC using a statistical method One-way ANOVA and statistical software GraphPad Prism. It can be seen that significant differences exist between the typical compounds represented by Formula I-1 to Formula I-48 and Formula II-1 to Formula II-144 and the PE group (P values are all less than 0.05).

**Table 1 Real-time quantitative PCR validation results of cardiomyocyte β-MHC (screening of compounds represented by Formula I)**

| No. | Mean | Statistical P Value |
|---|---|---|
| control group | 1.00 | 0.006 |
| PE group | 2.58 | - |
| PE+compound represented by Formula I-1 | 1.21 | P < 0.05 |
| PE+compound represented by Formula I-2 | 1.21 | P < 0.05 |
| PE+compound represented by Formula I-3 | 1.24 | P < 0.05 |
| PE+compound represented by Formula I-4 | 1.15 | P < 0.05 |
| PE+compound represented by Formula I-5 | 1.25 | P < 0.05 |
| PE+compound represented by Formula I-10 | 1.14 | P < 0.05 |
| PE+compound represented by Formula I-12 | 1.19 | P < 0.05 |
| PE+compound represented by Formula I-18 | 1.18 | P < 0.05 |
| PE+compound represented by Formula I-19 | 1.26 | P < 0.05 |
| PE+compound represented by Formula I-21 | 1.05 | P < 0.05 |
| PE+compound represented by Formula I-27 | 1.27 | P < 0.05 |
| PE+compound represented by Formula I-28 | 1.07 | P < 0.05 |
| PE+compound represented by Formula I-31 | 1.29 | P < 0.05 |
| PE+compound represented by Formula I-33 | 1.22 | P < 0.05 |
| PE+compound represented by Formula I-38 | 1.26 | P < 0.05 |
| PE+compound represented by Formula I-40 | 1.28 | P < 0.05 |
| PE+compound represented by Formula I-41 | 1.37 | P < 0.05 |
| PE+compound represented by Formula I-45 | 1.39 | P < 0.05 |
| PE+compound represented by Formula I-48 | 1.58 | P < 0.05 |
| Note: the statistically significant P value is the result of comparing each group with the PE group, and PE refers to phenylephrine. | | |

| **Table 2 Real-time quantitative PCR validation results of cardiomyocyte β-MHC** (screening of compounds represented by Formula II) | | |
|---|---|---|
| No. | Mean | Statistical P Value |
| control group | 1.00 | - |
| PE group | 2.49 | - |
| PE+compound represented by Formula II-1 | 1.32 | P < 0.05 |
| PE+compound represented by Formula II-2 | 1.42 | P < 0.05 |
| PE+compound represented by Formula II-3 | 1.28 | P < 0.05 |
| PE+compound represented by Formula II-7 | 1.15 | P < 0.05 |
| PE+compound represented by Formula II-9 | 1.18 | P < 0.05 |
| PE+compound represented by Formula II-10 | 1.27 | P < 0.05 |
| PE+compound represented by Formula II-17 | 1.13 | P < 0.05 |
| PE+compound represented by Formula II-18 | 1.19 | P < 0.05 |
| PE+compound represented by Formula II-25 | 1.52 | P < 0.05 |
| PE+compound represented by Formula II-26 | 1.42 | P < 0.05 |
| PE+compound represented by Formula II-30 | 1.21 | P < 0.05 |
| PE+compound represented by Formula II-36 | 1.41 | P < 0.05 |
| PE+compound represented by Formula II-42 | 1.48 | P < 0.05 |
| PE+compound represented by Formula II-43 | 1.21 | P < 0.05 |
| PE+compound represented by Formula II-49 | 1.36 | P < 0.05 |
| PE+compound represented by Formula II-54 | 1.28 | P < 0.05 |
| PE+compound represented by Formula II-57 | 1.26 | P < 0.05 |
| PE+compound represented by Formula II-63 | 1.36 | P < 0.05 |
| PE+compound represented by Formula II-64 | 1.22 | P < 0.05 |
| PE+compound represented by Formula II-78 | 1.35 | P < 0.05 |
| PE+compound represented by Formula II-82 | 1.15 | P < 0.05 |
| PE+compound represented by Formula II-90 | 1.27 | P < 0.05 |
| PE+compound represented by Formula II-91 | 1.57 | P < 0.05 |
| PE+compound represented by Formula II-95 | 1.24 | P < 0.05 |
| PE+compound represented by Formula II-96 | 1.38 | P < 0.05 |
| Note: the statistically significant P value is the result of comparing each group with the PE group, and PE refers to phenylephrine. | | |

**Table 3 Real-time quantitative PCR validation results of cardiomyocyte β-MHC (screening of compounds represented by Formula II)**

| No. | Mean | Statistical P Value |
|---|---|---|
| control group | 1.00 | - |
| PE group | 2.42 | - |
| PE+compound represented by Formula II-102 | 1.28 | P < 0.05 |
| PE+compound represented by Formula II-104 | 1.25 | P < 0.05 |
| PE+compound represented by Formula II-110 | 1.34 | P < 0.05 |
| PE+compound represented by Formula II-116 | 1.58 | P < 0.05 |
| PE+compound represented by Formula II-120 | 1.45 | P < 0.05 |
| PE+compound represented by Formula II-121 | 1.25 | P < 0.05 |
| PE+compound represented by Formula II-128 | 1.27 | P < 0.05 |
| PE+compound represented by Formula II-129 | 1.34 | P < 0.05 |
| PE+compound represented by Formula II-133 | 1.46 | P < 0.05 |
| PE+compound represented by Formula II-142 | 1.40 | P < 0.05 |
| PE+compound represented by Formula II-144 | 1.39 | P < 0.05 |
| Note: the P value is the result of comparing each group with the PE group, and PE refers to phenylephrine. | | |

The cardiomyocyte area was directly measured and the differences between the groups were compared for verification. The cardiomyocytes were co-intervened with PE and drugs and placed under a microscope for direct observation, and images were taken. The cell area data was calculated using Image J software. The results are shown in Table 4. The results show that the cardiomyocyte areas of the typical drug intervention groups of the two structures represented by Formula I and Formula II are both significantly less than that of the PE group, while Formula I-24-A, Formula I-24-B and Formula I-24-C have no apparent improvement effect.

**Table 4**

| Compound No. | Drug Concentration: 10 µM | Statistical P Value |
|---|---|---|
| | Cell Area Hypertrophy Multiple (Mean±Standard Deviation) Compared with Control Wells | |
| | | (Compared with PE Group) |
| PE | 1.91±0.14 | |
| Formula I-1 | 1.42±0.16 | P < 0.05 |
| Formula I-2 | 1.18±0.16 | P < 0.05 |
| Formula I-3 | 1.07±0.15 | P < 0.05 |
| Formula I-4 | 1.27±0.34 | P < 0.05 |
| Formula I-5 | 1.50±0.15 | P < 0.05 |
| Formula I-24 | 1.36±0.12 | P < 0.05 |
| Formula I-48 | 1.06±0.08 | P < 0.05 |
| Formula I-79 | 1.15±0.16 | P < 0.05 |
| Formula I-86 | 1.37±0.11 | P < 0.05 |
| Formula I-95 | 1.41±0.12 | P < 0.05 |
| Formula II-1 | 1.39±0.30 | P < 0.05 |
| Formula II-2 | 1.33±0.11 | P < 0.05 |
| Formula II-3 | 1.28±0.10 | P < 0.05 |
| Formula II-7 | 1.14±0.14 | P < 0.05 |
| Formula II-48 | 1.07±0.20 | P < 0.05 |
| Formula II-73 | 1.17±0.16 | P < 0.05 |
| Formula II-96 | 1.49±0.10 | P < 0.05 |
| Formula II-121 | 1.48±0.09 | P < 0.05 |
| Formula II-144 | 1.12±0.13 | P < 0.05 |
| Formula I-24-A | 1.77±0.11 | P > 0.05 |
| Formula I-24-B | 1.81±0.20 | P > 0.05 |
| Formula I-24-C | 1.95±0.08 | P > 0.05 |

### Example 197 Pharmacodynamic Test of Models of Heart Failure due to Pressure Overload of Mice

### 1 Materials and method

1.1 Animals healthy C57BL/6 mice

### 1.2 Drug and administration regimen

| **Group** | **Compound Treatment** | **Dosage (mg/kg)** | **Route of Administration** | **Frequency of Administration/Period** |
|---|---|---|---|---|
| 1 | modeling group | -- | intraperitoneal | once a day for four consecutive weeks |
| 2 | modeling+drug | 5 | intraperitoneal | once a day for four consecutive weeks |

### 1.5 Method

### Modeling

Modeling by thoracic aorta ligation surgery: preoperative echocardiography was performed to exclude mice with abnormal cardiac functions; for mice with normal cardiac functions, anesthesia was performed, and the mice were anesthetized and then transferred into a small animal operating table and fixed; an open airway was established via endotracheal intubation, and anesthesia was maintained; taking the second rib as the center, the skin was cut along the sternum toward the first and third ribs to expose the connection between the second rib and the sternum; the second rib was cut to expose the aorta thoracic segment; the aortic arch was freed, a thin thread was inserted underneath, a thin needle was placed in parallel to the aortic arch for co-ligation; after ligation, the thin needle was withdrawn to observe the condition of the mice, and the mice were sutured layer by layer after stabilization; the mice were placed on the thermal insulation pad to keep warm, and after waking up, the mice were put back into the cage for daily feeding according to the activity.

### Experimental grouping and administration regimen

After five weeks of modeling, echocardiography was performed on the mice to determine the ejection fractions of the hearts of the mice. When the ejection fraction decreased by about 50%, the ejection fraction met the experimental requirements, and the administration experiment started. Animals were grouped randomly and administered according to the ultrasound data, the echocardiography was performed every two weeks until week 9 ended, and pathological section analysis was performed.

### Statistical analysis

The results were expressed using mean±standard error, data analysis was performed using GraphPad Prism statistical software, and the comparison was analyzed using a t-test. P < 0.05 was determined that there was a difference with statistical significance, otherwise P was determined that there was no significant difference.

### 2 Results

### 2.1 Effect of test drugs on disease development in mice

The results are shown in Table 5 and Table 6. It can be seen that after four weeks of administration (nine weeks of modeling), the typical drugs represented by Formula I and Formula II both have a significant improvement effect on the cardiac contractile function, while Formula I-24-A, Formula I-24-B and Formula I-24-C have no apparent improvement effect.

**Table 5**

| Compound No. | Ejection Fraction (Mean) of Ventriculus Sinister of Heart | Statistical P Value (Compared with Model Group) |
|---|---|---|
| model group | 28.6% | - |
| Formula I-1 | 37.2% | P < 0.05 |
| Formula I-2 | 38.3% | P < 0.05 |
| Formula I-3 | 37.8% | P < 0.05 |
| Formula I-4 | 35.4% | P < 0.05 |
| Formula I-5 | 39.7% | P < 0.05 |
| Formula I-24 | 40.0% | P < 0.05 |
| Formula I-48 | 37.2% | P < 0.05 |
| Formula I-24-A | 29.4% | P > 0.05 |
| Formula I-24-B | 27.2% | P > 0.05 |
| Formula I-24-C | 30.7% | P > 0.05 |

**Table 6**

| Compound No. | Ejection Fraction (Mean) of Heart | Statistical P Value (Compared with Model Group) |
|---|---|---|
| model group | 29.8% | - |
| Formula II-1 | 38.7% | P < 0.05 |
| Formula II-2 | 37.3% | P < 0.05 |
| Formula II-3 | 40.1% | P < 0.05 |
| Formula II-7 | 39.6% | P < 0.05 |
| Formula II-48 | 41.4% | P < 0.05 |
| Formula II-73 | 40.5% | P < 0.05 |
| Formula II-96 | 36.8% | P < 0.05 |
| Formula II-121 | 42.2% | P < 0.05 |
| Formula II-144 | 41.5% | P < 0.05 |

After four weeks of administration (nine weeks of modeling), the hearts of the mice were subjected to tissue sampling. The pathological results show that the cardiac fibrosis is significantly increased after the modeling treatment and the degree of fibrosis is significantly reduced after the treatment of the typical test drugs (described in Table 5 and Table 6) represented by Formula I and Formula II in various groups.

Based on the preceding examples of the present application, the benzimidazole compound of the present application has a short synthesis process, and the synthesis process is easy to control; the benzimidazole compound of the present application has a myocardial protective effect, can be used for treating chronic heart failure diseases and has a broad application prospect.

The applicant has stated that although the compound, the preparation method therefor and the use thereof in the present application are described through the preceding embodiments, the present application is not limited to the preceding embodiments, which means that the implementation of the present application does not necessarily depend on the preceding embodiments. It should be apparent to those skilled in the art that any improvements made to the present application, equivalent replacements of raw materials selected in the present application and additions of adjuvant ingredients thereof, and selections of specific methods, etc., all fall within the protection scope and the disclosed scope of the present application.

## Claims

1. A benzimidazole compound having a structure represented by the following Formula A: 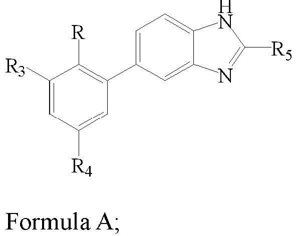
wherein R is hydroxyl or 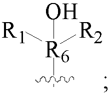
R₁, R₂, R₃ and R₅ are each independently hydrogen, halogen, nitro, amino, hydroxyl, cyano, carboxyl, C1-C4 linear or branched alkoxyformyl, aminoformyl, aminoformyl substituted on N with C1-C4 linear or branched alkyl, C1-C4 linear or branched alkyl, C1-C4 alkoxy and C1-C4 alkylamino;
R₄ is fluorine, bromine, nitro, hydroxyl, cyano, carboxyl, C1-C4 linear or branched alkoxyformyl, aminoformyl, aminoformyl substituted on N with C1-C4 linear or branched alkyl, C1-C4 linear or branched alkyl, non-halogenated C1-C4 alkoxy or C1-C4 alkylamino;
R₆ is C1-C4 linear or branched alkylene; and
in a case where R₄ is carboxyl, R₃ is hydrogen and R is hydroxyl, R₅ is not methyl.

2. The benzimidazole compound according to claim 1, wherein the benzimidazole compound has a structure represented by Formula I or Formula II: 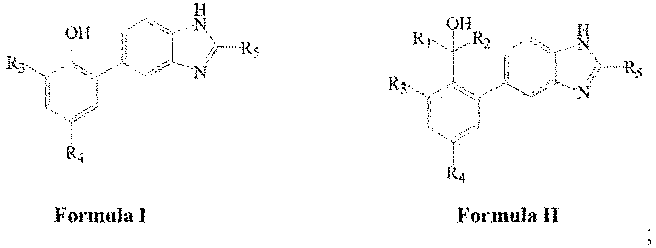 wherein R₁, R₂, R₃, R₄ and R₅ are defined in a same manner as those in Formula A.

3. The benzimidazole compound according to claim 1 or 2, wherein R₁, R₂, R₃ and R₅ are each independently any one of hydrogen, fluorine, chlorine, bromine, amino, hydroxyl, cyano, methoxyformyl, ethoxyformyl, dimethylaminoformyl, diethylaminoformyl, trifluoromethyl, methyl, ethyl, *n*-propyl, isopropyl, allyl, cyanomethyl, methoxy, trifluoromethoxy, ethoxy, methylamino, dimethylamino, ethylamino or diethylamino;
preferably, R₄ is any one of fluorine, bromine, amino, hydroxyl, cyano, carboxyl, methoxyformyl, ethoxyformyl, aminoformyl, dimethylaminoformyl, diethylaminoformyl, trifluoromethyl, methyl, ethyl, *n*-propyl, isopropyl, allyl, cyanomethyl, methoxy, ethoxy, methylamino, dimethylamino, ethylamino or diethylamino;
preferably, R₁ and R₂ are hydrogen or methyl;
preferably, R₃ is cyano, fluorine or hydrogen;
preferably, R₄ is fluorine, allyl, *n*-propyl, cyanomethyl, carboxyl, methoxyformyl, aminoformyl or dimethylaminoformyl; and
preferably, R₅ is hydrogen or methyl.

4. The benzimidazole compound according to any one of claims 1 to 3, wherein the benzimidazole compound is any one of compounds represented by the following Formula I-1 to Formula I-47 and Formula II-1 to Formula II-144:

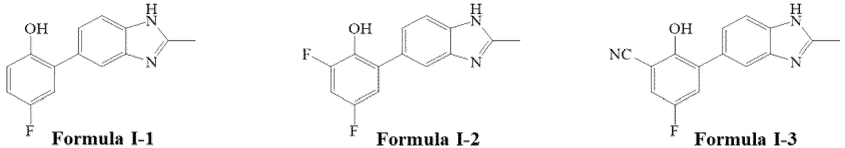

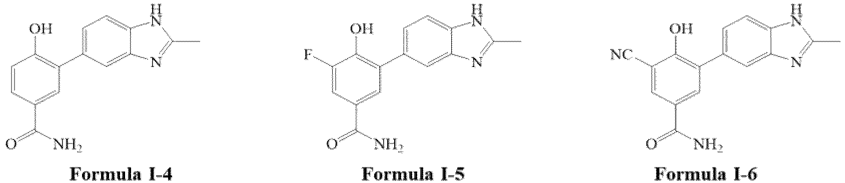

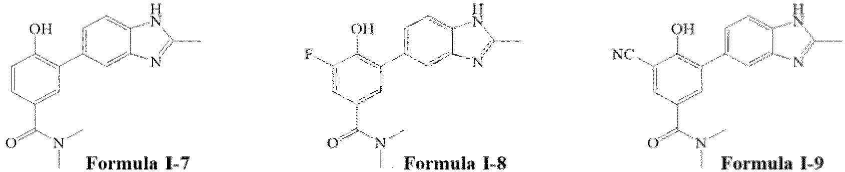

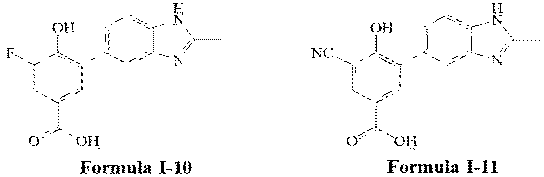

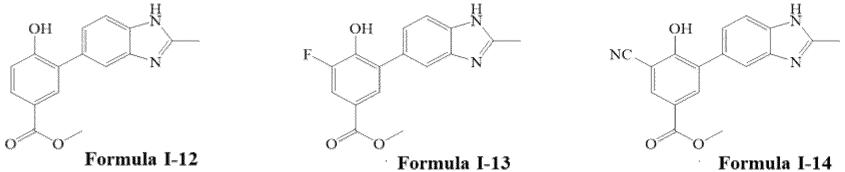

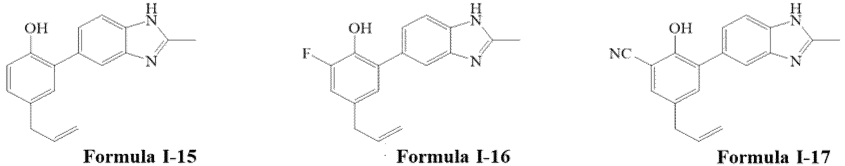

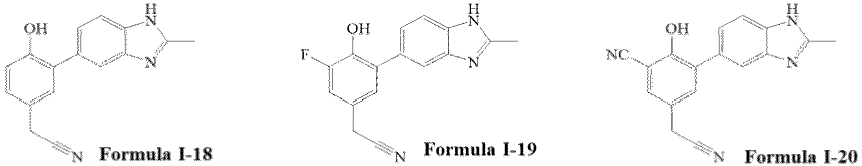

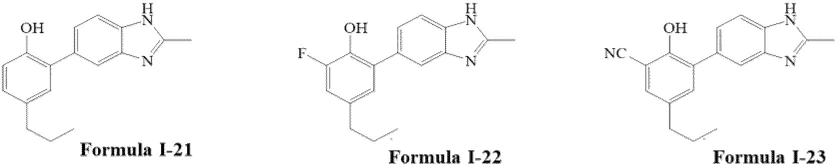

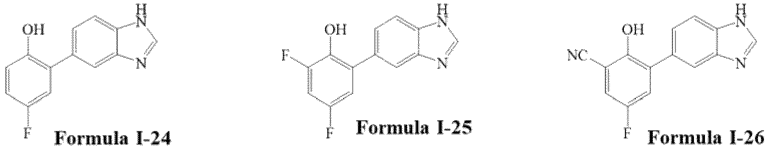

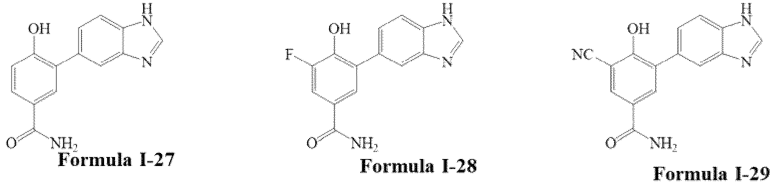

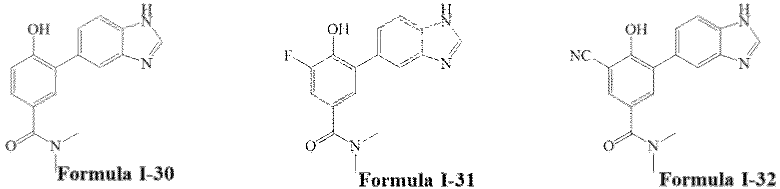

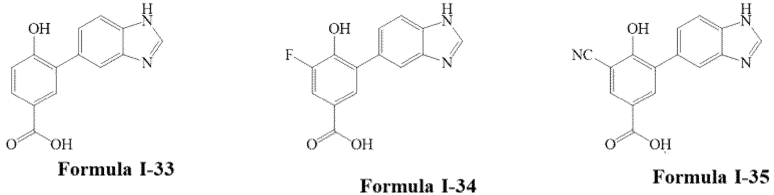

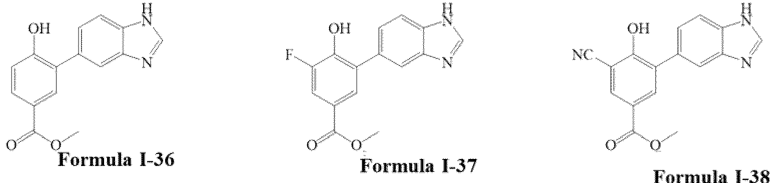

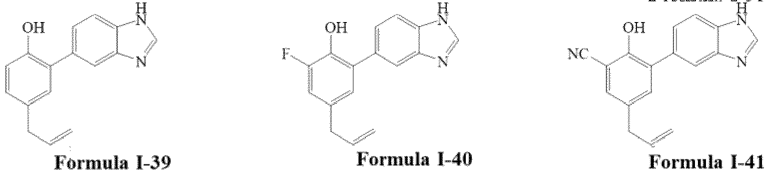

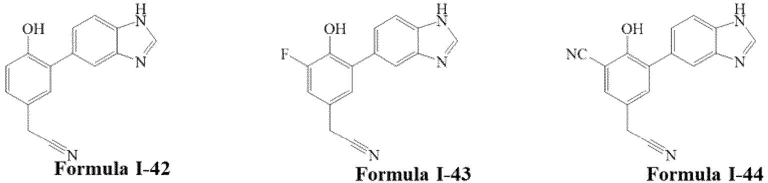

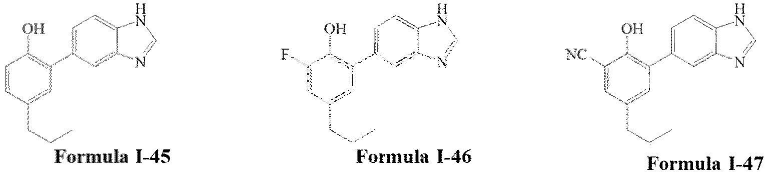

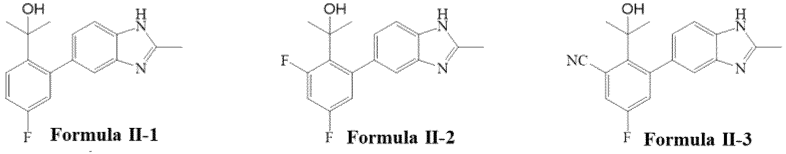

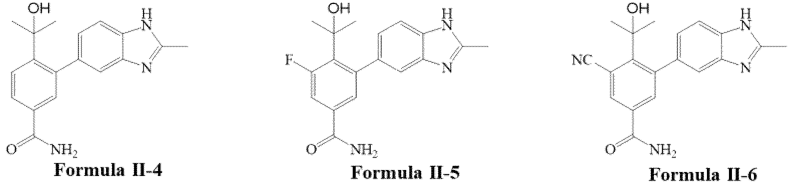

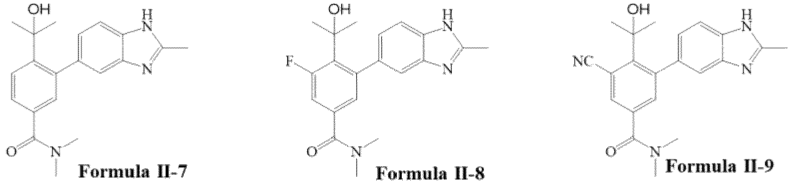

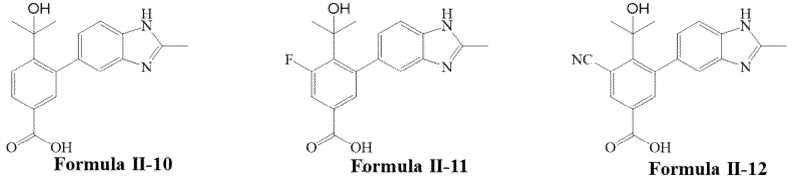

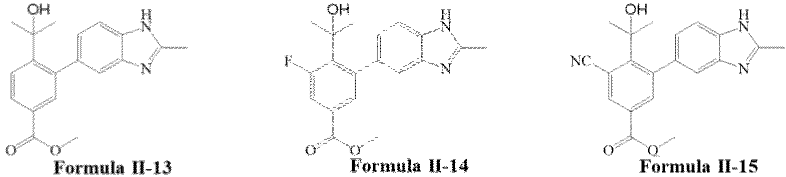

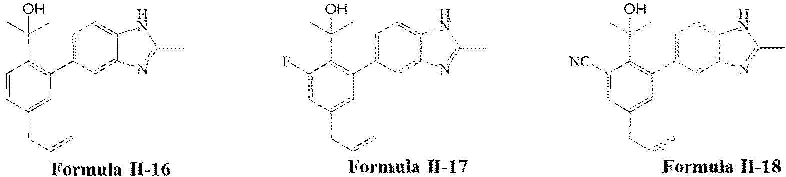

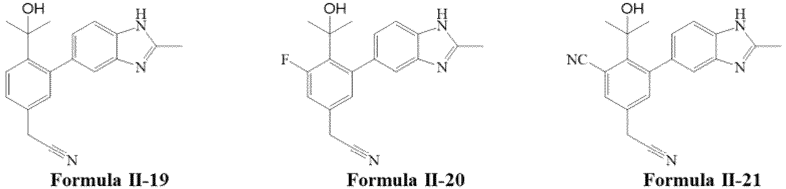

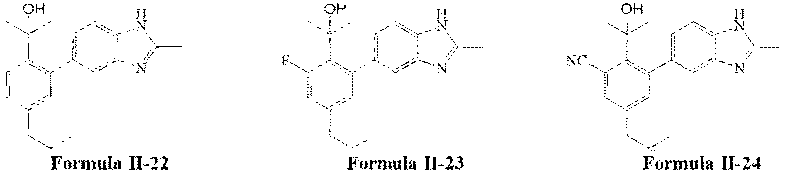

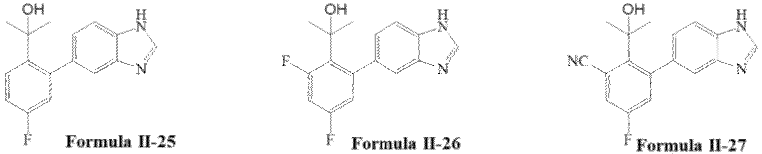

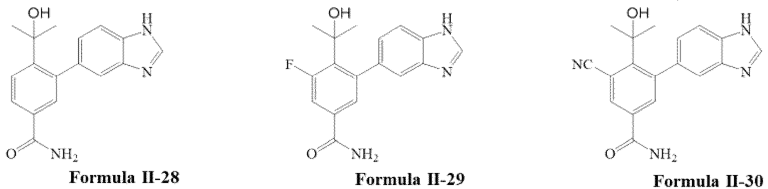

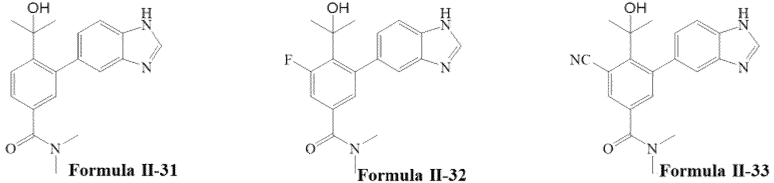

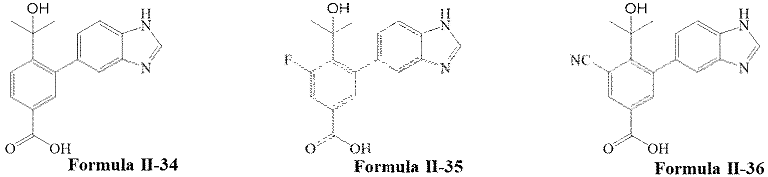

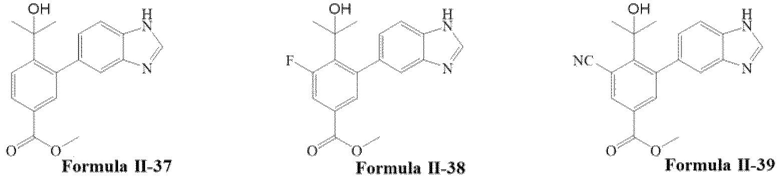

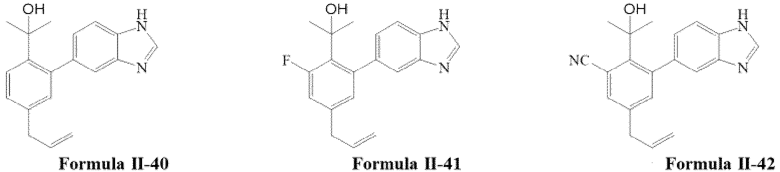

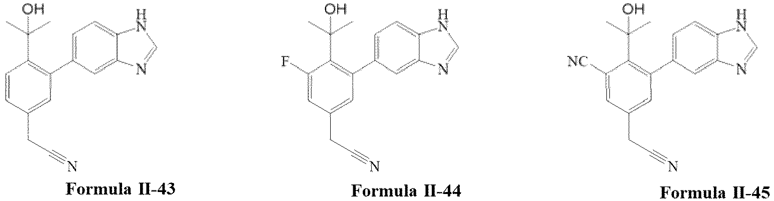

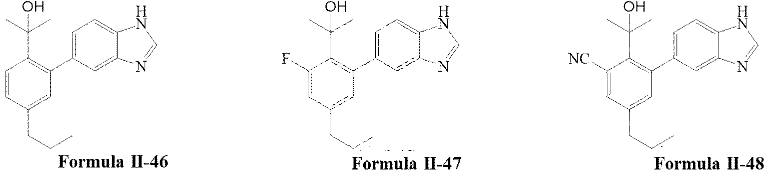

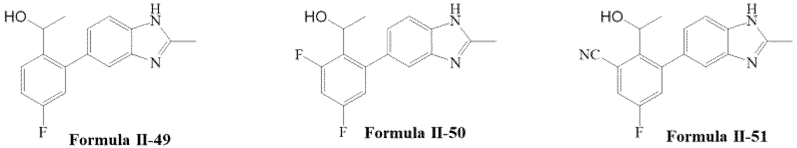

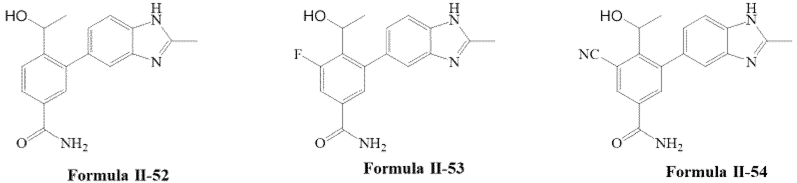

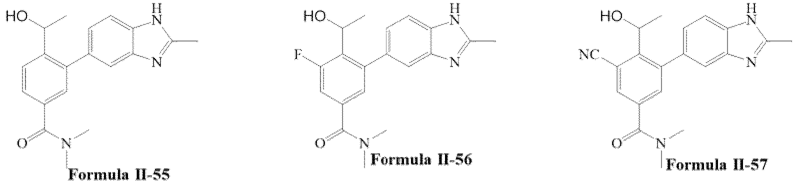

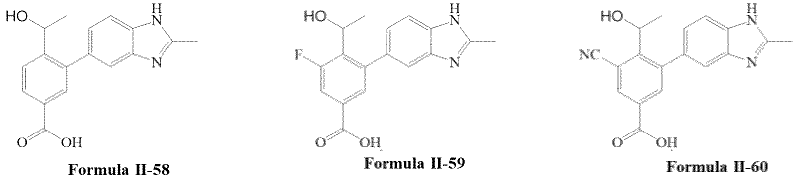

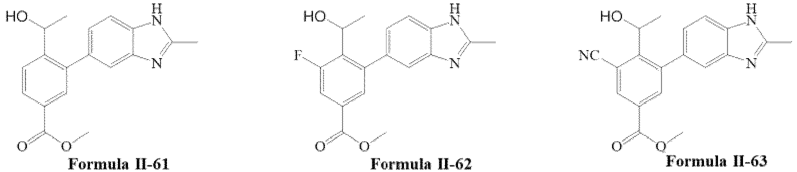

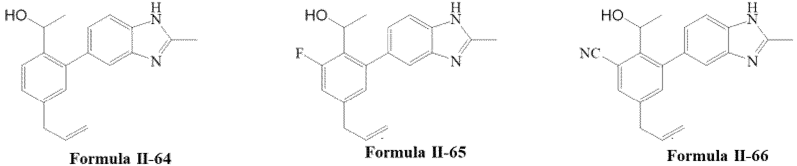

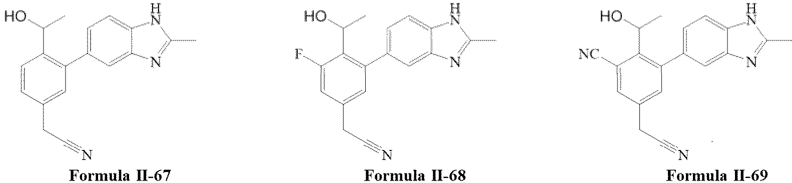

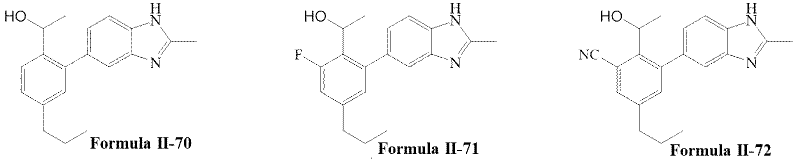

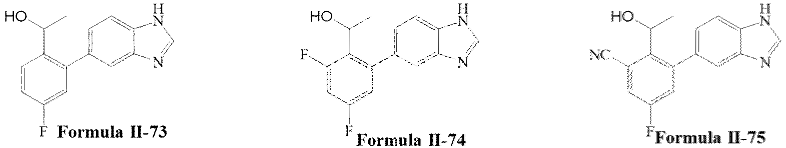

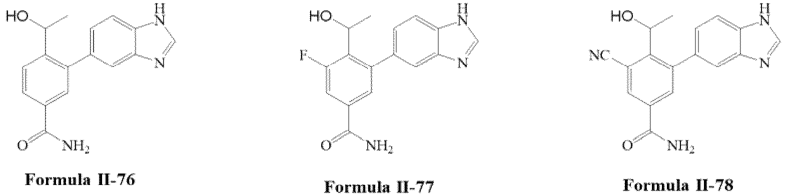

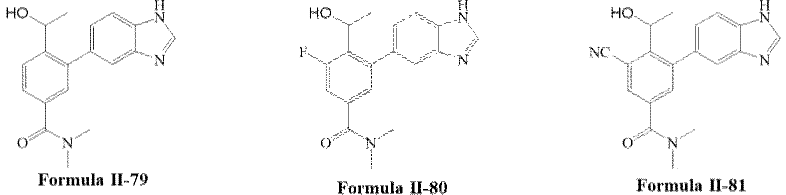

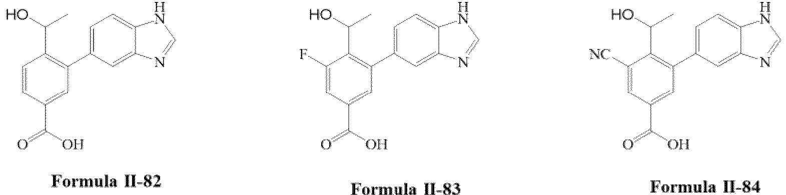

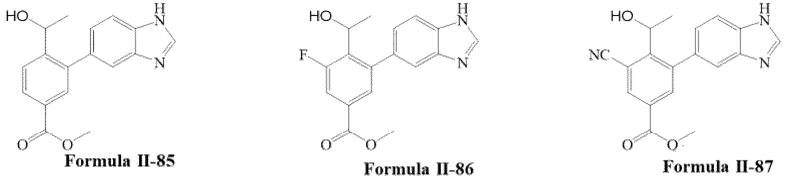

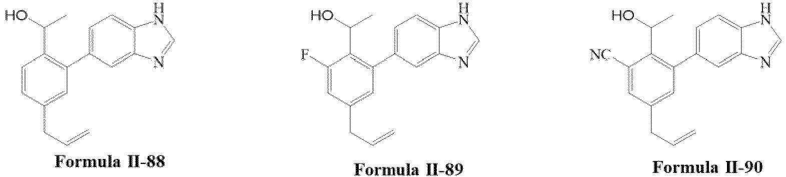

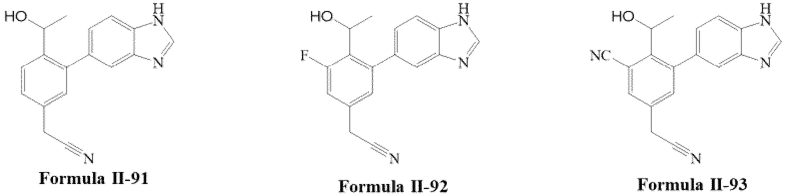

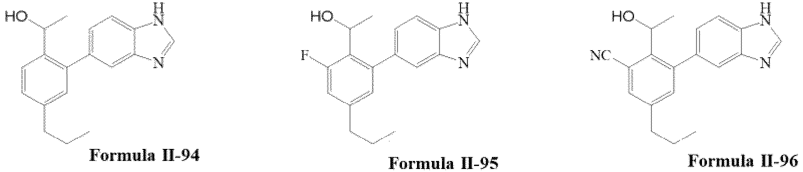

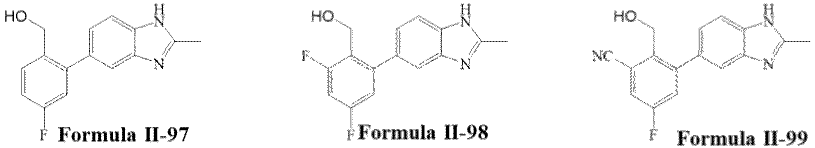

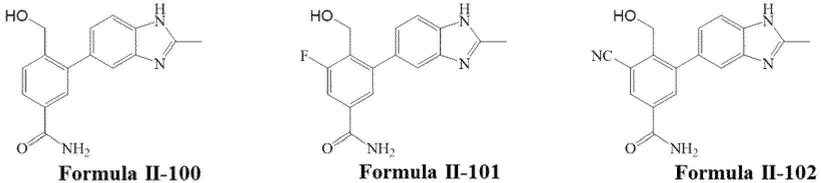

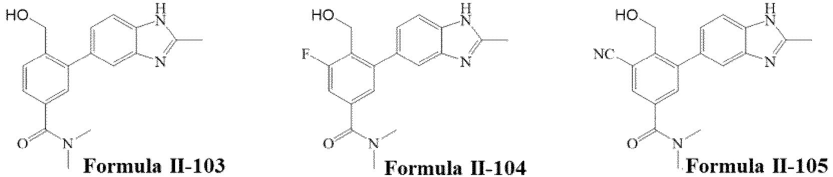

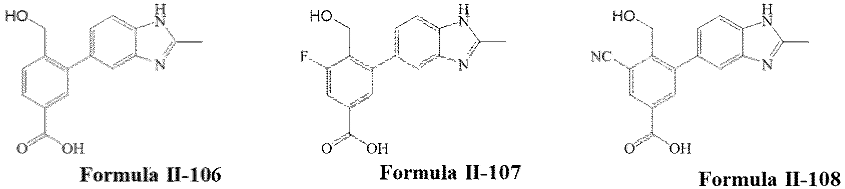

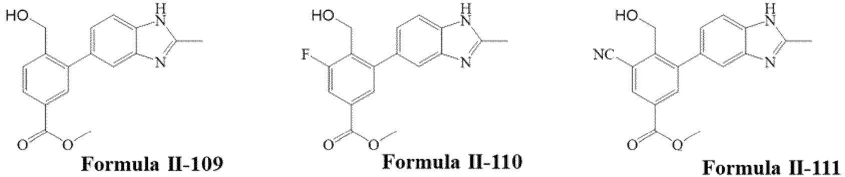

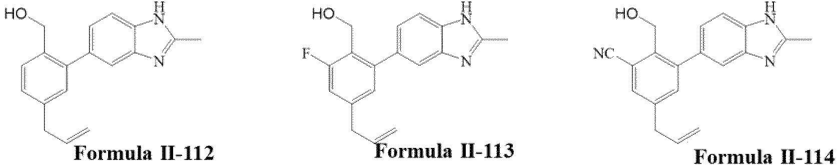

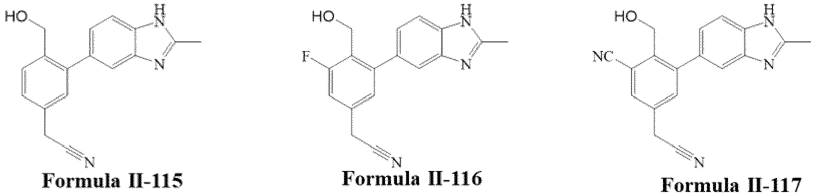

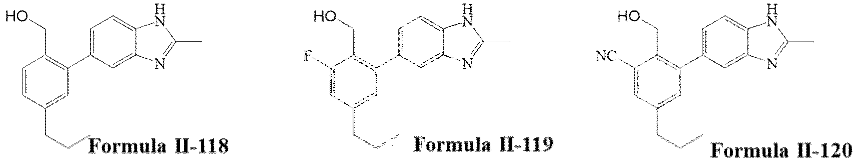

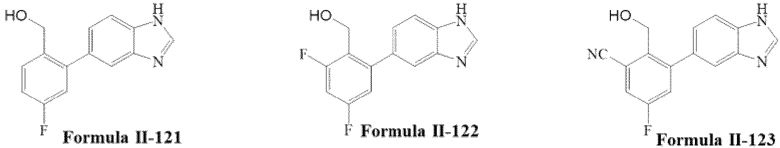

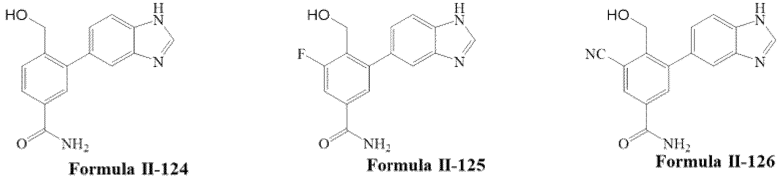

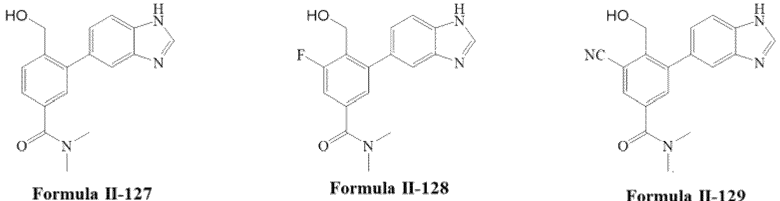

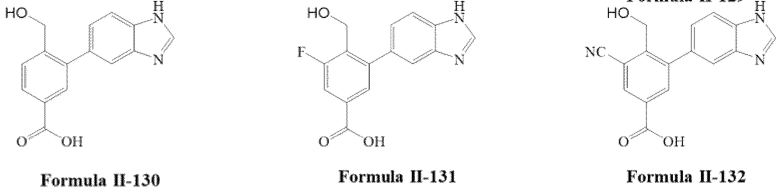

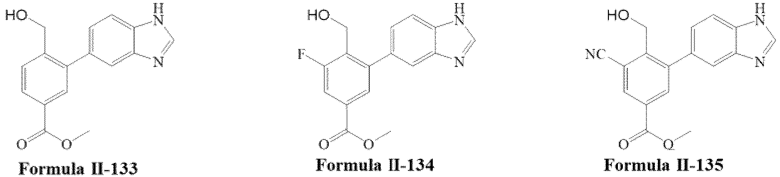

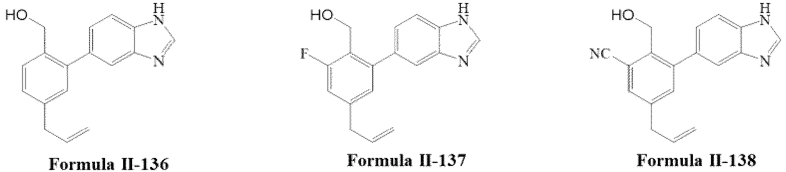

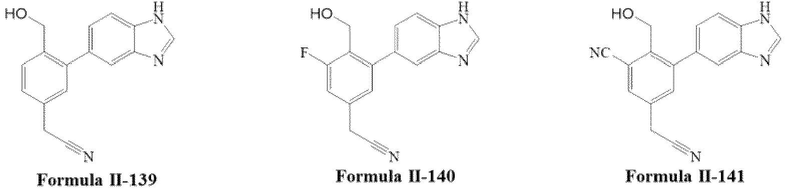

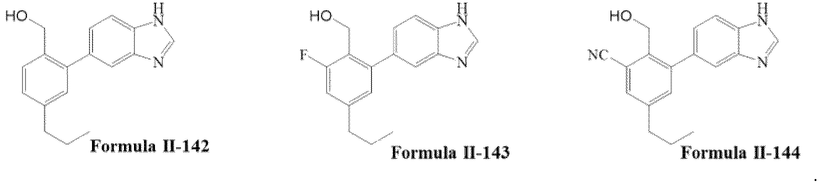

5. A method for preparing the benzimidazole compound according to any one of claims 1 to 4, comprising the following step: performing a Suzuki reaction on a brominated compound represented by Formula B and a boronic acid compound represented by Formula V under an action of a catalyst to obtain the benzimidazole compound represented by Formula A with the following reaction formula: 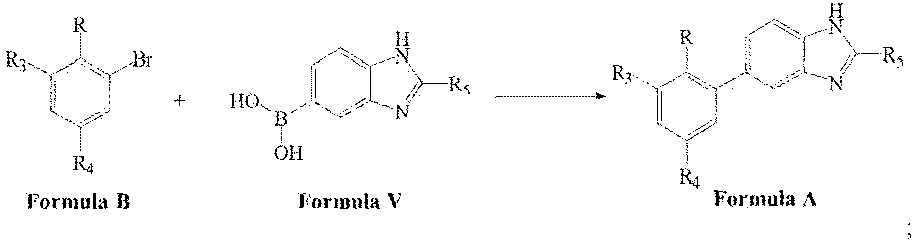
wherein preferably, a molar ratio of the brominated compound represented by Formula B to the boronic acid compound represented by Formula V is 1:0.8 to 1:3;
preferably, the catalyst is any one or a combination of at least two of tetrakis(triphenylphosphine)palladium, [1,1-bis(diphenylphosphino)ferrocene]palladium(II) dichloride or palladium acetate;
preferably, a molar ratio of the catalyst to the boronic acid compound represented by Formula V is 0.001:1 to 0.5:1;
preferably, the Suzuki reaction is conducted in the presence of an alkaline substance;
preferably, the alkaline substance is any one or a combination of at least two of potassium fluoride, potassium acetate, sodium carbonate, potassium carbonate or potassium phosphate;
preferably, the Suzuki reaction is conducted in an organic solvent, and the organic solvent is any one or a combination of at least two of DMF, toluene, ethanol, 1,4-dioxane, water or THF; and
preferably, the Suzuki reaction is conducted for a period of 0.25-48 h at a temperature of 70-150 °C.

6. A pharmaceutically acceptable salt of the benzimidazole compound according to any one of claims 1 to 4;
wherein preferably, the pharmaceutically acceptable salt is a salt of an organic acid or a salt of an inorganic acid of the benzimidazole compound;
preferably, the salt of an organic acid is selected from any one of tartrate, stearate, oxalate, citrate, lactate, sorbate, fumarate, formate, acetate, benzoate, benzenesulfonate, ethanesulfonate, resinate, trifluoroacetate, maleate, malate, L-malate, methanesulfonate, fumarate, an amino acid salt or nicotinate;
preferably, the salt of an organic acid is selected from any one of tartrate, acetate, maleate, malate, L-malate or fumarate;
preferably, the salt of an inorganic acid is selected from any one of phosphate, sulfate, nitrate, iodate, bromate, hydroiodide, hydrobromide or hydrochloride; and
preferably, the salt of an inorganic acid is selected from any one of phosphate, sulfate or hydrochloride.

7. A solvate, a prodrug, a tautomer or a stereochemical isomer of the benzimidazole compound according to any one of claims 1 to 4;
wherein preferably, the solvate is a hydrate and/or an alcoholate of the benzimidazole compound.

8. A pharmaceutical composition, comprising the benzimidazole compound according to any one of claims 1 to 4;
wherein preferably, the pharmaceutical composition further comprises a pharmaceutically acceptable adjuvant;
preferably, the pharmaceutically acceptable adjuvant is any one of an excipient, a diluent, a carrier, a flavoring agent, a binder or a filler; and
preferably, the pharmaceutical composition is in a dosage form of an oral preparation, a parenteral preparation or an external-application preparation.

9. Use of the benzimidazole compound according to any one of claims 1 to 4, or the pharmaceutically acceptable salt of the benzimidazole compound according to claim 6, or the solvate, the prodrug, the tautomer or the stereochemical isomer according to claim 7 or the pharmaceutical composition according to claim 8 for preparing a drug for treating chronic heart failure.

10. Use of a substituted benzimidazole compound, a tautomer of the substituted benzimidazole compound, a pharmaceutically acceptable salt of the substituted benzimidazole compound, a solvate of the substituted benzimidazole compound, a hydrate of the substituted benzimidazole compound or a pharmaceutical composition of the substituted benzimidazole compound for preparing a drug for treating chronic heart failure;
wherein the substituted benzimidazole compound has the following structure:
